# EUROPEAN PATENT SPECIFICATION

(11) **EP 2 442 667 B1**
(45) Date of publication and mention of the grant of the patent: **17.04.2013**
(21) Application number: 10728592.6
(22) Date of filing: 15.06.2010
(51) Int. Cl.: A23F 5/00, A23F 5/04, A23F 5/16, A23F 5/24, A23L 1/30, A61K 31/4425

(54) **NMP-ENRICHED EXTRACT**
NMP-ANGEREICHERTER EXTRAKT
EXTRAIT ENRICHI DE NMP

(30) Priority: 15.06.2009 EP 09007839
(43) Date of publication of application: 25.04.2012
(73) Proprietor: Tchibo GmbH, 22297 Hamburg (DE); Deutsche Forschungsanstalt für Lebensmittelchemie, 85748 Garching (DE); Technische Universität München, 85354 Freising (DE)
(72) Inventor: SOMOZA, Veronika, 85748 Garching (DE); HOFMANN, Thomas, 85354 Freising (DE); LANTZ, Ingo, 22297 Hamburg (DE); STIEBITZ, Herbert, 22297 Hamburg (DE); BYTOF, Gerhard, 22297 Hamburg (DE); LANG, Roman, 85354 Freising (DE); RUBACH, Malte, 80796 München (DE)
(74) Representative: Müller-Boré & Partner Patentanwälte
(86) International application number: PCT/EP2010/003576
(87) International publication number: WO 2010/145798

(56) References cited:
- STEGEN VAN DER G H D: "THE EFFECT OF DEWAXING OF GREEN COFFEE ON THE COFFEE BREW" FOOD CHEMISTRY, ELSEVIER SCIENCE PUBLISHERS LTD, GB, vol. 4, no. 1, 1 January 1979 (1979-01-01) , pages 23-29, XP000602966 ISSN: 0308-8146 cited in the application
- STADLER R H ET AL: "Alkylpyridiniums. 2. Isolation and Quantification in Roasted and Ground Coffee" JOURNAL OF AGRICULTURAL AND FOOD CHEMISTRY, AMERICAN CHEMICAL SOCIETY, WASHINGTON, US, vol. 50, no. 5, 27 February 2002 (2002-02-27), pages 1200-1206, XP002524075 ISSN: 0021-8561 [retrieved on 2002-02-02]

## Description

The present invention relates to a method for producing a N-methylpyridinium (NCMP)-enriched extract from trigonelline-containing organic material.

Extracts of trigonelline-containing organic material, like for example coffee beverages, are complex solutions containing bioactive compounds that interact with their micro-environment in the human stomach. Due to this interaction it is supposed that gastric acid secretion is enhanced. Therefore, gastric irritation after coffee consumption has been reported for several times. In the complex process of gastric acid secretion, the key player is the gastric H+, K+-ATPase. Due to activation of the parietal cell by hormones and transmitters it transports hydrogen into the gastric lumen in exchange to potassium and acidifies the stomach. In parallel, chloride is secreted into the gastric lumen. The whole process is regulated due to cell surface receptors. The stimulation of gastric acid secretion is mediated by the histamine H2 receptor, the acetylcholine M3 receptor and the cholecystocinin beta receptor. The counter-regulation takes place due to activation of the somatostatin receptor. Therefore, the hormones histamine, gastrin and somatostatin as well as acetylcholine play a crucial role in the regulation of acid secretion. The activation of cell surface receptors is directly connected with intracellular signal transduction. MAPK kinases and receptor tyrosine kinases have been shown to be involved in the regulation of gastric acid secretion. So far activation of epithelial growth factor receptor (EGFr) is related to an enhanced secretory status of the parietal cell. Subsequent signaling activates Akt1 and ERK1/2 MAPK kinases. Akt1 signaling activates transcription of the H+, K+-ATPase and also enhances the secretory activity of the parietal cell. ERK1/2 exhibited an acute inhibitory effect on the secretory activity, but it's chronic stimulation is supposed to be pro-secretory. However, ERK1/2 is also thought to activate gene expression of the H+, K+-ATPase. Apart from these signaling pathways cyclic AMP is formed after activation of GS-protein linked histamine H2 receptor activation. Cyclic AMP may activate protein kinase A which in turn can activate transcription factor in the nucleus. The transcription factor ATF-2 may be involved in the expression of the H+, K+-ATPase and of the somatostatin receptor 2 since both genes exhibit a cyclic AMP responsive element.

Before roasting, untreated coffee beans have about 1000 to 1400 µg/g N-alkanoyl-5-hydroxytryptamide (C5HT), roasted coffee beans have about 500 to 800 µg/g C5HT. Further, roasted coffee beans which have been decaffeinated have about 50 µg/l C5HT. Additionally, coffee containing caffeine has about 200 to 500 µg/l C5HT, whereas decaffeinated coffee has about 50 µg/l C5HT.

Accordingly, consumption of coffee has often been reported to be associated with heartburn or stomach irritation which can both be induced by an increased stomach acid secretion. The effect of coffee beverages on gastric irritation and the intra-gastric pH in humans was first studied by Ehrlich et al. (Ehrlich A, Basse H, Henkel-Ernst J, Hey B, Menthe J, and Lücker PW. Effect of differently processed coffee on the gastric activity difference and intragastric pH in healthy volunteers. Methods Find Exp Clin Pharmacol 20: 155-161, 2006.). After oral administration of 150 ml coffee beverage prepared from either a regular or a steam-treated coffee, the latter induced significantly less mucosal irritation in healthy volunteers in than the regular coffee beverage. Based on these results, steam-treatment of coffee was hypothesized to significantly reduce stomach-irritating compounds in roasted coffee beans and coffee manufacturers started to label steam-treated coffee as 'stomach-friendly'. This technology was initially developed to remove caffeine and chlorogenic acids as the main compounds alleviating the sensory qualities of coffee beverages.

Thus, the technical problem underlying the present invention is to provide a product or other means for the prevention or reduction of stomach problems caused by gastric acid secretion which might be used for example as a dietary supplement.

The solution to the above technical problem is achieved by the embodiments characterized in the claims.

In particular, the present invention relates to a method for producing a N-methylpyridinium (NMP)-enriched extract from trigonelline-containing organic material, the method comprising the steps of:
(a) dewaxing the trigonelline-containing organic material;
(b) roasting the trigonelline-containing organic material;
(c) treating the roasted trigonelline-containing organic material with hot water to obtain an aqueous extract; and
(d) adding NMP to the aqueous extract.

The dewaxing of the trigonelline-containing organic material, the roasting of the trigonelline-containing organic material as well as the hot water extraction can be carried out by methods well known in the art.

For example, the dewaxing in step (a) can be carried out by the methods described in van der Stegen, 1979 (van der Stegen, The effect of dewaxing of green coffee on the coffee brew, Fd. Chem (4) 23-29, 1979).

When the trigonelline-containing organic material contains caffeine, the method of the present invention may optionally further comprise the step of decaffeination of the trigonelline-containing organic material before and/or after step (a), for example by treating the trigonelline-containing organic material with ethyl acetate. This step can be carried out by methods well known in the art.

In a preferred embodiment, the roast degree of the trigonelline-containing organic material after the roasting in step (b) is at least 50, more preferred at least 80, more preferred at least 100 scale parts. In another preferred embodiment, the roast degree of the trigonelline-containing organic material after the roasting in step (b) is from 40 to 110 scale parts, more preferred from 60 to 90 scale parts, and even more preferred from 70 to 90 scale parts, and most preferred from 70 to 80 scale parts. Regarding the color, a roast degree of less than 50 scale parts is considered to be dark, a roast degree of about 75 scale parts is considered to be medium, and a roast degree of at least 90 scale parts is considered to be light. The roast degree can be easily determined by a person skilled in the art, for example by using the color detection device Dr. Lange - LFM 1, the color detection device Dr. Lange - LK 100, or the color detection device RSM 2 produced by Schaltex GmbH, according to the respective protocols provided by the manufacturers of said devices.

After the roasting in step (b) of the above method, the trigonelline-containing organic material preferably has a C5HT content of less than 50 µg/g C5HT, more preferably less than 40 µg/g C5HT, even more preferably less than 30 µg/g C5HT, even more preferably less than 20 µg/g C5HT, and most preferably less than 10 µg/g C5HT.

The method according to the present invention optionally further comprises the step of grinding the roasted trigonelline-containing organic material after step (b). In a preferred embodiment of the present invention, the trigonelline-containing organic material is coffee which is ground to a grading selected from the group consisting of fine, medium, and crude.

The treatment with hot water in step (c) is not subject to any limitation. For example, the treatment with hot water in step (c) can be carried out for at least 30 seconds and/or the water can have a temperature of at least 80°C.

The method of the present invention optionally further comprises the steps of filtering the aqueous extract prior to and/or after step (d). Preferably, the filtering is carried out at room-temperature using commercially available coffee filters via gravity force.

In a further preferred embodiment, at least 2.95 mg/100 ml NMP, more preferably at least 5 mg/100 ml NMP, more preferably at least 8 mg/100 ml NMP are added to the aqueous extract in step (d).

Preferably, the NMP-enriched extract obtained by the method according to present invention contains at least 23 mg/L NMP, more preferably at least 36 mg/L NMP, more preferably at least 48 mg/L NMP.

The NMP-enriched extract obtained by the method according to the present invention is preferably C5HT-depleted. In particular, the NMP-enriched extract obtained by the method according to the present invention is preferably CSHT-depleted relative to the trigonelline-containing organic material used as starting material in the method according to the present invention.

In a preferred embodiment, the NMP-enriched extract obtained by the method according to the present invention has less than 50 µg/l C5HT, more preferably less than 40 µg/l C5HT, even more preferably less than 30 µg/l C5HT, even more preferably less than 20 µg/l C5HT, and most preferably less than 10 µg/l C5HT.

In another preferred embodiment, the ratio NMP/C5HT x 100 in the NMP-enriched extract obtained by the method according to present invention is at least 100, more preferably at least 120, more preferably at least 140, and most preferably at least 180.

The term "N-methylpyridinium" as used herein relates to N-methylpyridinium in its ionic form, as well as in salt-form, for example as its iodide (NMPI), chloride, hydroxide or sulphate. The abbreviations N-MP and NMP are used herein synonymously for N-methylpyridinium.

The term "trigonelline-containing organic material" as used herein relates to any naturally occurring material that contains the alkaloid trigonelline.

In general, the trigonelline-containing organic material is selected from the group consisting of coffee, in particular *Coffea arabica, Coffea canephora, Coffea spp.,* and *Psilanthus spp.,* members of the *Fabaceae,* in particular *Pisum sativum, Glycine max, Phaseolus vulgaris, Lens culinaris, Cicer arietinum,* and *Trigonella foenum-graecum,* members of the *Chenopodiaceae,* in particular *Chenopodium quinoa,* and members of the *Poaceae,* in particular *Avena sativa.*

In a preferred embodiment, the trigonelline-containing organic material is *Coffea spp.* In a more preferred embodiment, the trigonelline-containing organic material is *Coffea arabica,* more preferably *Coffea arabica, provenience Columbia-* or *Coffea arabica, provenience Brazil.* In another preferred embodiment, the trigonelline-containing organic material is *Coffea canephora* also known as "robusta coffee", more preferably *Coffea canephora, provenience Vietnam.*

Said N-methylpyridinium (NMP)-enriched extract can be used as a dietary supplement.

In the course of the present invention, there has been developed foodstuff that is enriched with N-methylpyridinium (NMP) by the addition of said NMP-enriched extract obtained by the present invention.

The term "foodstuff' as used herein relates to any substance that can be eaten or drunk by an animal or a human for example for nutrition and/or pleasure. In an especially preferred embodiment, the foodstuff is coffee.

Advantageously, the NMP-enriched extract from trigonelline-containing organic material obtained by the method according to the present invention combines a high NMP content with a low C5HT content. By dewaxing the trigonelline-containing organic material the NMP content of the extract is maintained and the C5HT content is depleted. Accordingly, the NMP-enriched extract has a very advantageous NMP/C5HT ratio. As a consequence, the anti-secretory effect regarding gastric acid of NMP is prolonged over time after consumption of the NMP-enriched extract obtained by the present invention by the minimization of the C5HT content.

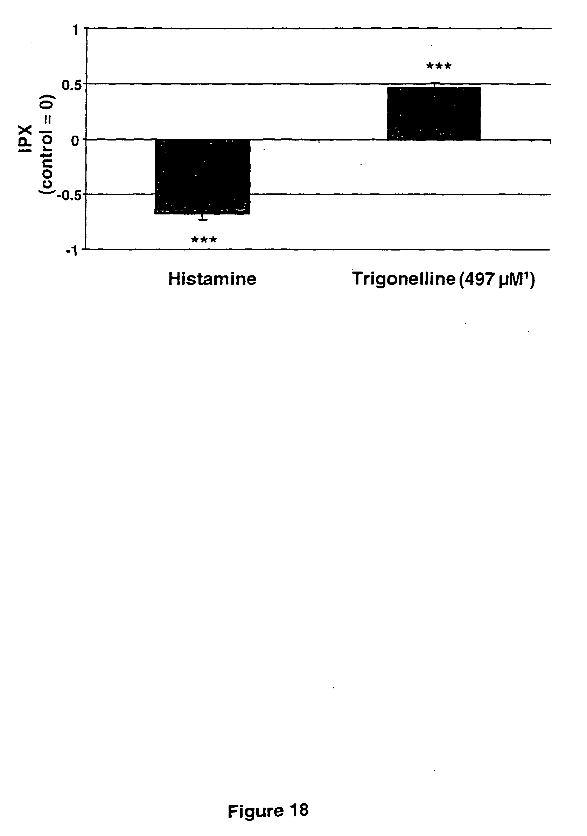

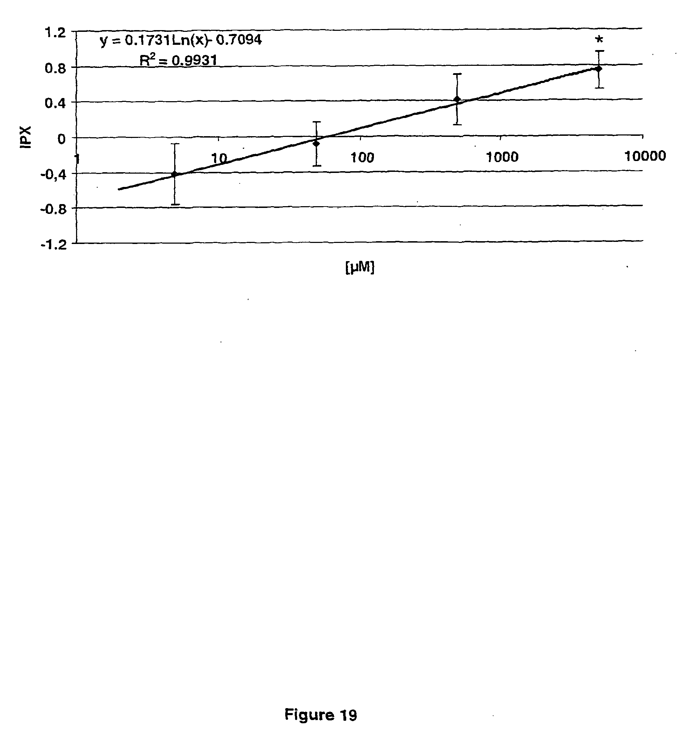

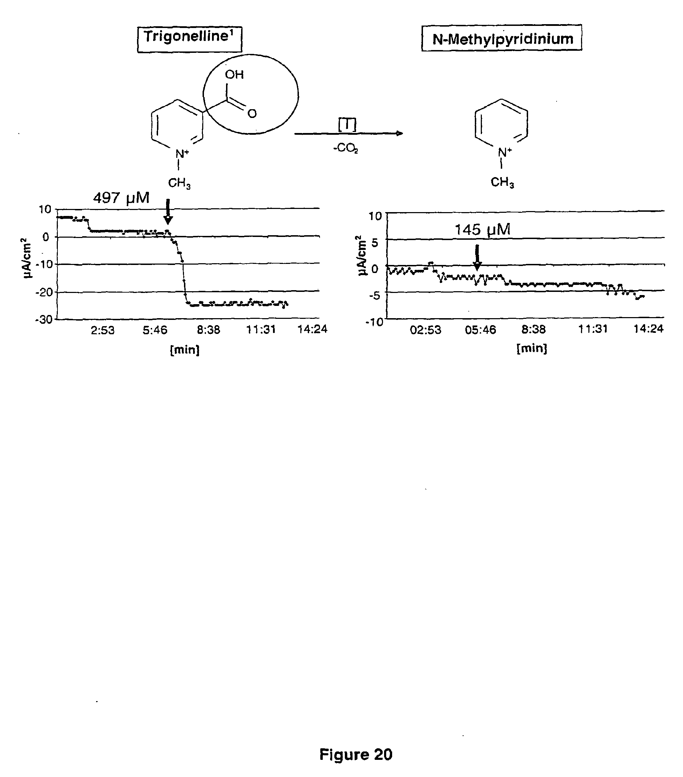

The figures show:

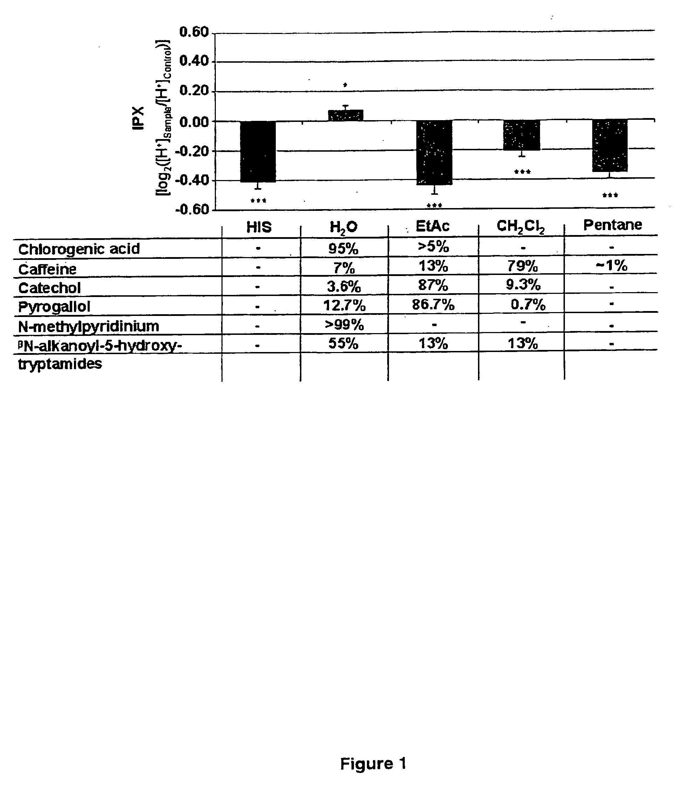

Figure 1: Intracellular proton index (IPX) of HGT-1 cells treated for 10 minutes with histamine (HIS, 1 mmol/L) or one of the solvent fractions prepared from a coffee beverage in concentrations according to their quantitative yields: water (H2O: 2.14 mg/mL), ethyl acetate (EtAc: 0.16 mg/mL), dichloromethane (CH2Cl2: 0.18 mg/mL) and pentane: 0.005 mg/mL). Distribution of quantitated compounds over the solvent fraction is given in the table below (Statistics: two-tailed t-test vs. control cells, ***=p.0.001, n=9).

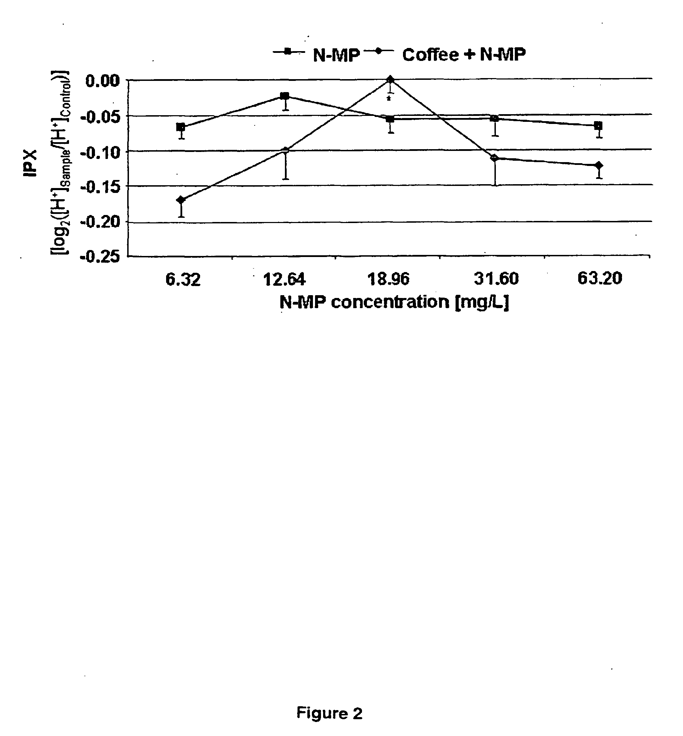

Figure 2: Intracellular proton index (IPX) of HGT-1 cells treated for 10 minutes with varying concentrations of N-methylpyridinium either as single compound (N-MP) or in combination with a lyophilizate prepared from a regular coffee (Coffee + N-MP), reaching the same N-methylpyridinium concentration in the system as were applied with the single compound (Statistics: one-way ANOVA with Holm-Sidak post-hoc test, *=p.0.05, n=9).

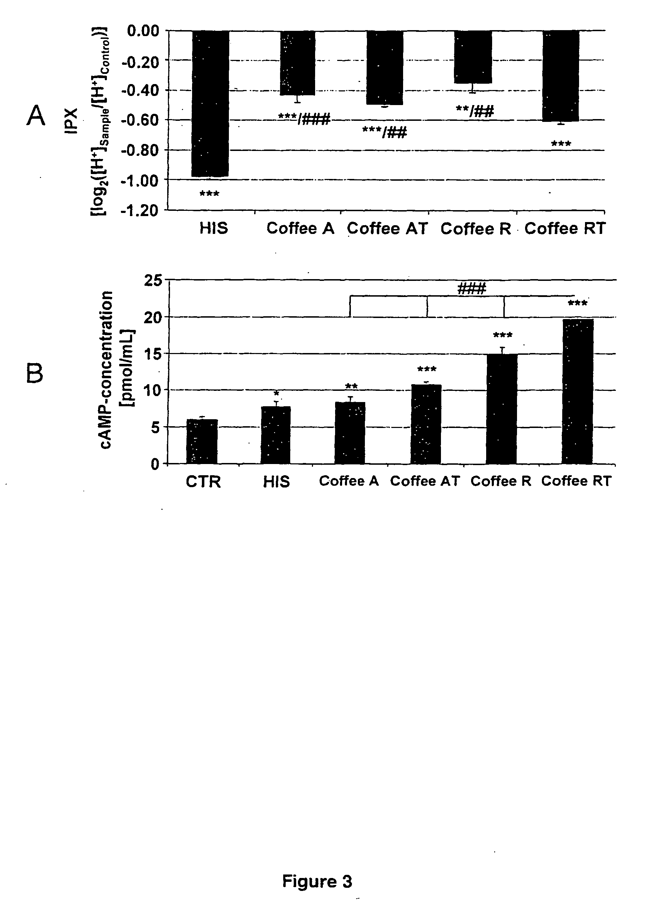

Figure 3: Intracellular proton index (IPX, exposure time: 10 min, A) and contents of cyclic AMP (cAMP, exposure time: 0.5 min, B) of HGT-1 cells treated with either histamine (HIS: 1 mmol/L) or lyophilizates (2.5 mg/mL) prepared from either C. Arabica Brazil (Coffee A), C. Arabica Brazil steam-treated (Coffee AT), C. Robusta Vietnam (coffee R) or C. Robusta Vietnam steam-treated (coffee RT), having high (coffee A, AT), medium (coffee R) and low (coffee RT) concentrations of N-methylpyridinium, respectively (Statistics: two-tailed t-test vs. control cells, **=p.0.01, ***=p.0.001, n=9; or vs. coffee RT ##=p.0.01, ###=p.0.001).

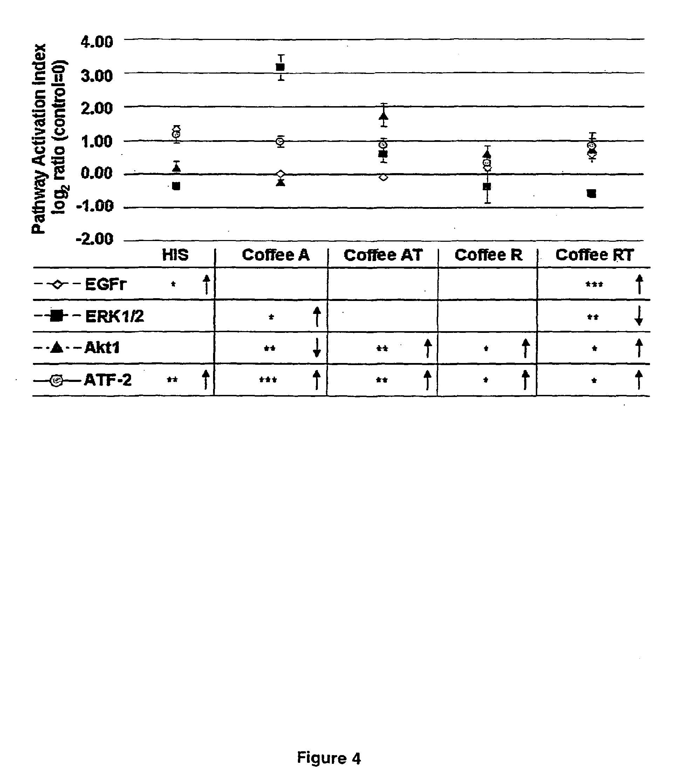

Figure 4: Pathway activation indices of EGFr, ERK1/2, Akt1 and ATF-2 in HGT-1 cells treated for 10 min with either histamine (HIS: 1 mmol/L) or lyophilizates (2.5 mg/mL) prepared from either C. Arabica Brazil (Coffee A), C. Arabica Brazil steam-treated (Coffee AT), C. Robusta Vietnam (coffee R) or C. Robusta Vietnam steam-treated (coffee RT), having high (coffee A, AT), medium (coffee R) and low (coffee RT) concentrations of N-methylpyridinium, respectively. (Statistics: two-tailed t-test vs. control cells, **=p.0.01, ***=p.0.001, n=9; or vs. coffee RT ##=p.0.01, ###=p.0.001).

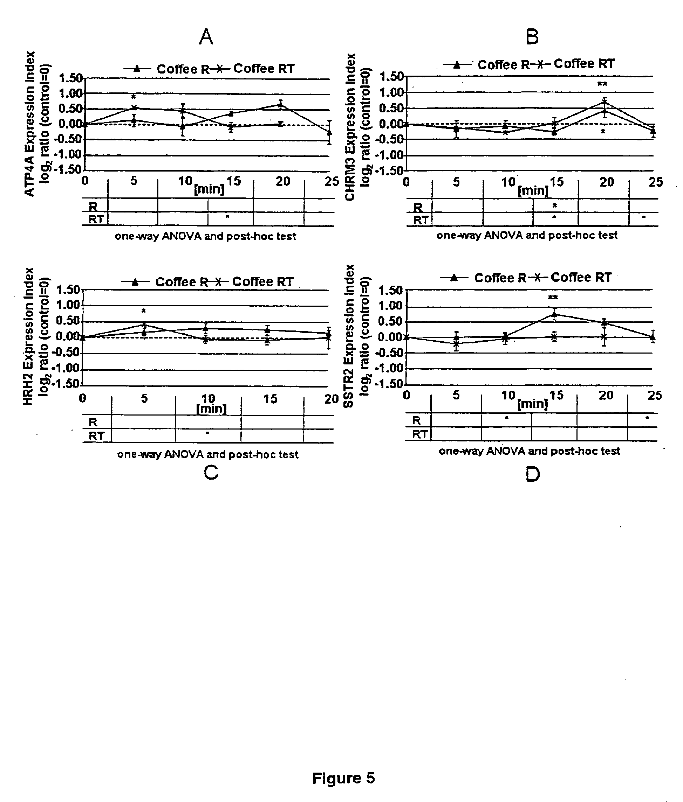

Figure 5: Time dependent indices of gene expression for the H+,K+-ATPase (ATP4A), the histamine H2 receptor (HRH2), the acetylcholine receptor M3 (CHRM3) and the somatostatin receptor (SSTR2) in HGT-1 cells after exposure of lyophilizates (2.5 mg/l) prepared from either C. Robusta Vietnam (coffee R) or C. Robusta Vietnam steam-treated (coffee RT), having medium (coffee R) and low (coffee RT) concentrations of N-methylpyridinium, respectively (Statistics: one-way ANOVA with Holm-Sidak post-hoc test, *=p.0.05, **=p.0.01, n=6; the p-value of the one-way ANOVA is given in the diagram, the tables below indicate the significant changes in the time course compared to the most pronounced change of expression identified by the post-hoc analysis).

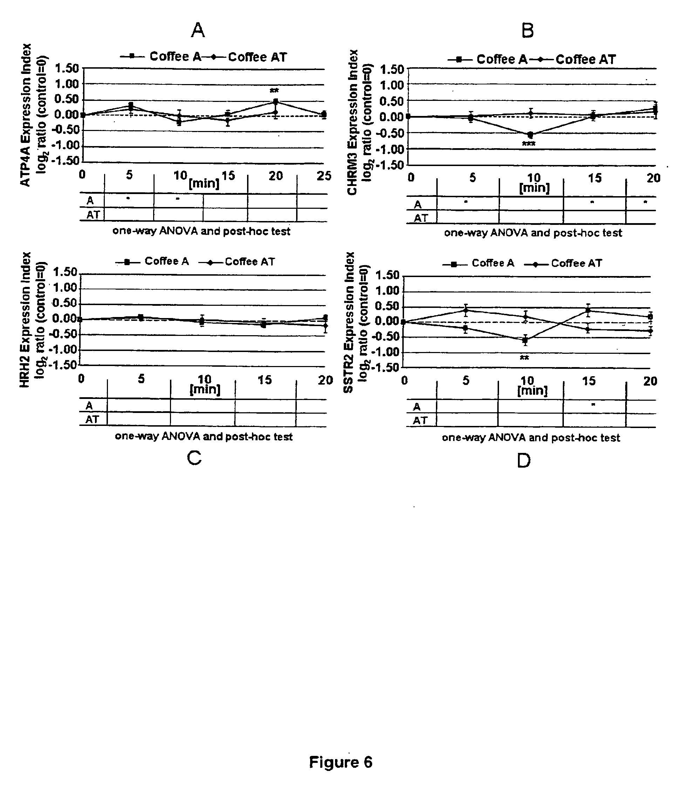

Figure 6: Time dependent indices of gene expression for the H+,K+-ATPase (ATP4A), the histamine H2 receptor (HRH2), the acetylcholine receptor M3 (CHRM3) and the somatostatin receptor (SSTR2) in HGT-1 cells after exposure of lyophilizates (2.5 mg/l) prepared from either C. Arabica Brazil (coffee A) or C. Arabica Brazil team-treated (coffee AT), having high concentrations of N-methylpyridinium (Statistics: one-way ANOVA with Holm-Sidak post-hoc test, *=p.0.05, **=p.0.01, n=6; the p-value of the one-way ANOVA is given in the diagram, the tables below indicate the significant changes in the time course compared to the most pronounced change of expression identified by the post-hoc analysis).

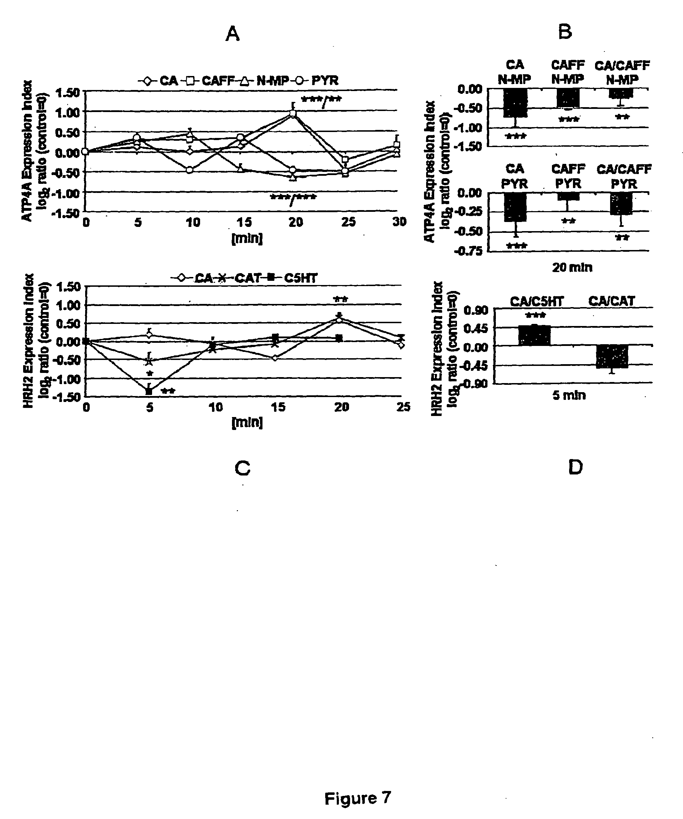

Figure 7: Effect of single compounds and combined compounds on the gene expression of the H+, K+-ATPase (ATP4A) and histamine receptor H2 (HRH2). A: The expression of ATP4A was tested to be up-regulated by chlorogenic acid (CA) and caffeine (CAFF) after 20 minutes. In contrast, pyrogallol (PYR) and N-methylpyridinium (N-MP) down-regulated the expression after 20 minutes. (Statistics: one-way ANOVA with post-hoc test). B: Combination of either CA, CAFF or both with N-NMP resulted in significant compensation of up-regulation by CAFF or CA (Statistics: t-test for CS or CF vs. combination with N-MP or PYR). C: N-alkanoyl-hydroxytryptamides (C5HT) tremendously down-regulated HRH2 after five minutes. Catechol (CAT) also caused a down-regulation. CA up-regulated HRH2 after 20 minutes. (Statistics: one-way ANOVA with post-hoc test). D: The combination of CA with C5HT turned out in a significantly increased expression of HRH2 (p.0.001) compared to control cells. CA combined with CAT did not alter the expression of HRH2 compared to CAT alone (Statistics: t-test for treated cells vs. control cells). (*=p.0.05, **=p.0..01, ***=p.0.001, n=5-9;)

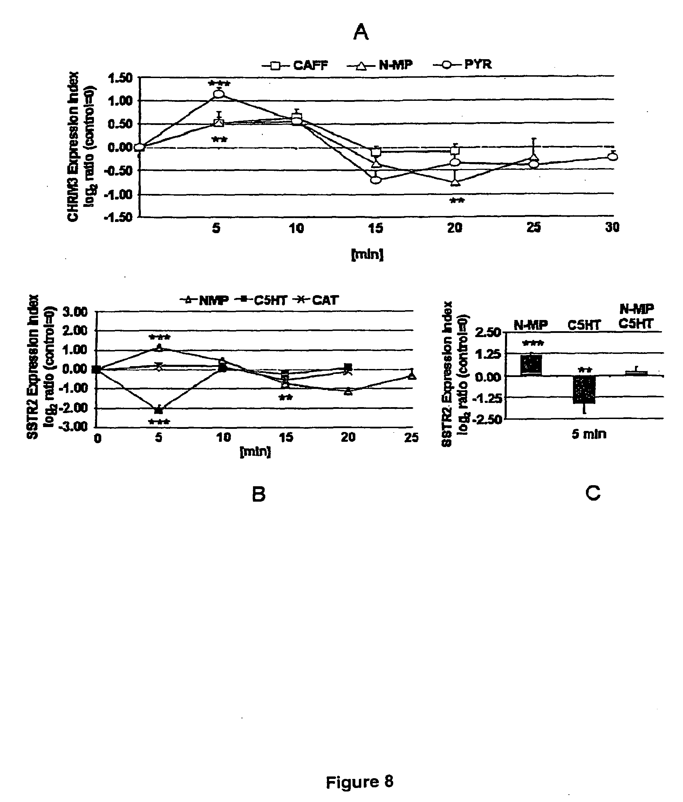

Figure 8: Effect of single compounds and combined compounds on the gene expression of the acetylcholine receptor M3 (CHRM3) and the somatostatin receptor 2 (SSTR2). A: The expression of CHRM3 was significantly increased by pyrogallol (PYR) and more slightly by caffeine (CAFF) and N-methylpyridinium (N-MP). N-MP caused subsequent down-regulation after 20 minutes as well as PYR after 15 minutes. (Statistics: one-way ANOVA with post-hoc test). B: The somatostatin receptor 2 was tremendously down-regulated by N-alkanoyl-hydroxytryptamides (C5HT) and slightly by catechol (CAT). N-MP up-regulated this anti-secretory receptor by more than two-fold with subsequent down-regulation. (Statistics: one-way ANOVA with post-hoc test). C: The combined effect of N-MP and C5HT after five minutes resulted in fully compensation of the effects of each single compound (Statistics: t-test for N-MP/C5HTvs. N-MP or C5HT). (*=p.0.05, **=p.0.01, ***=p.0.001, n=5-9;)

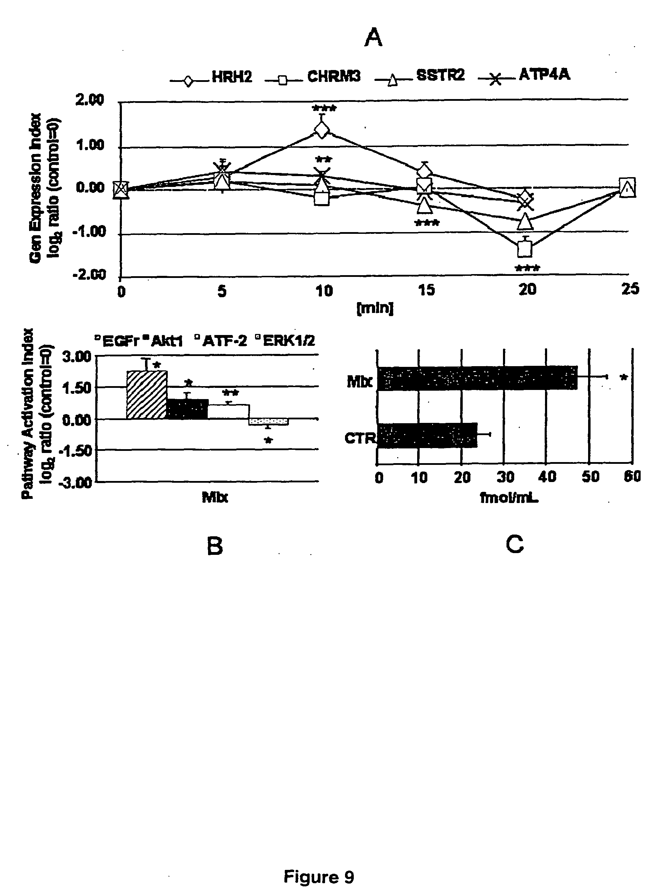

Figure 9: Effect of the full recombination of all compounds on the gene expression of the histamine receptor H2 (HRH2) the acetylcholine receptor M3 (CHRM3), the somatostatin receptor 2 (SSTR2) and the H+, K+-ATPase (ATP4A). Also presented are signal transduction activation of kinases and the transcription factor ATF-2 as well as cyclic AMP concentrations. A: HRH2 was strongly up-regulated by the mix of all compounds after 10 minutes. CHRM3 and SSTR2 were down-regulated with a minimum after 20 minutes. ATP4A was regulated toa smaller extent than the receptors, but still significant at five and 10 minutes. (Statistics: one-way ANOVA with post-hoc test). B: EGFr was significantly activated by the mix of all compounds as well as Akt1 and the transcription factor ATF-2. In, contrast the phosphorylation status of ERK1/2 was shown to be below control levels. (t-test for treated cells vs. non-treated cells (CTR)). C: Cyclic AMP concentrations were increased by two-fold after treatment with the compound mix for one minute. (t-test for treated cells vs. non-treated cells (CTR)). (*=p.0.05, **=p.0.01, ***=p.0.001, n=3-9;)

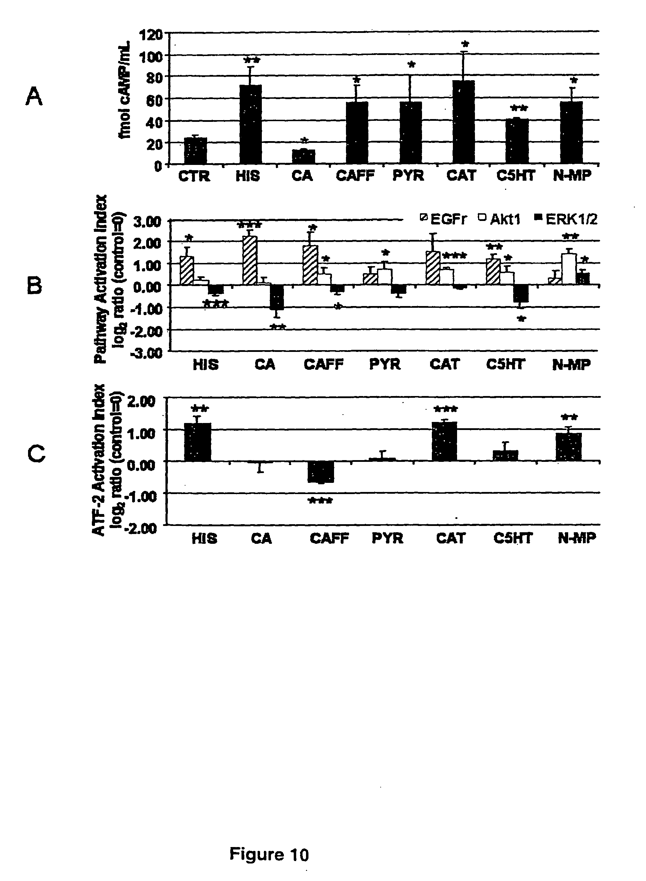

Figure 10: Influence of single compounds on cyclic AMP concentrations, activation of kinases and the transcription factor ATF-2. A: Histamine (HIS), caffeine (CAFF), catechol (CAT), pyrogallol (PYR), N-alkanoyl-hydroxytryptamides (C5HT) and N-methylpyridinium (N-MP) increased cyclic AMP concentrations significantly compared to control cells (CTR). Chlorogenic acid (CA) caused significant decrease .of the cyclic AMP concentrations. B: Epidermal growth factor receptor (EGFr) was significantly activated by HIS, CA, CAFF and C5HT, but not by PYR, CAT and N-MP. Akt1 kinase was only significantly activated by treatment with CAFF, PYR, CAT, C5HT and N-MP. ERK1/2 signaling was solely increased due to treatment with N-MP. C: The cyclic AMP responsive element binding protein, known as transcription factor ATF-2, was significantly activated by HIS, CAT and N-MP. CAFF caused an activation status below control levels. (Statistics: t-test for treated cells vs. non-treated cells (CTR), *=p.0.05, **=p.0.01, ***=p.0.001, n=3-7)

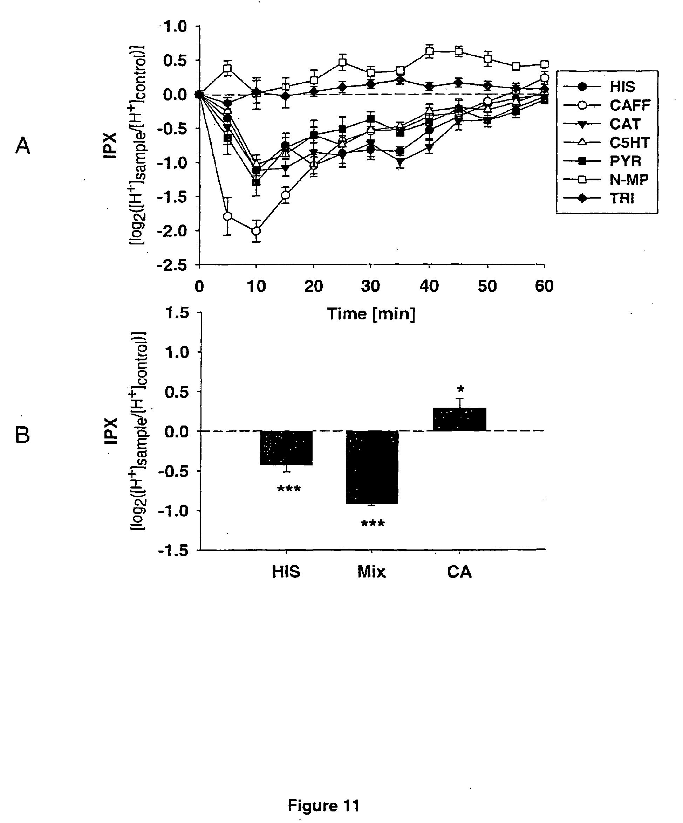

Figure 11: Measurement of secretory activity by the intracellular proton index (IPX). A: Treatment of HGT-1 cells with histamine (HIS), caffeine (CAFF), pyrogallol (PYR), catechol (CAT) or C5HT resulted in increased secretion indicated by a negative IPX. In contrast, chlorogenic acid (CA) N-methylpyridinium (N-MP) and trigonelline (TRI) caused a decreased proton secretion indicated by a positive IPX. Statistics: one-way ANOVA, p=0,01.0,001; n=10-12). B: Treatment of HGT-1 cells with chlorogenic acid (CA) increased the current whereas a recombination of all compounds (Mix) caused a decreased IPX. (Statistics: t-test for treated cells vs. non-treated cells, **=p=0.05, ***=p=0.001, n=3-7)

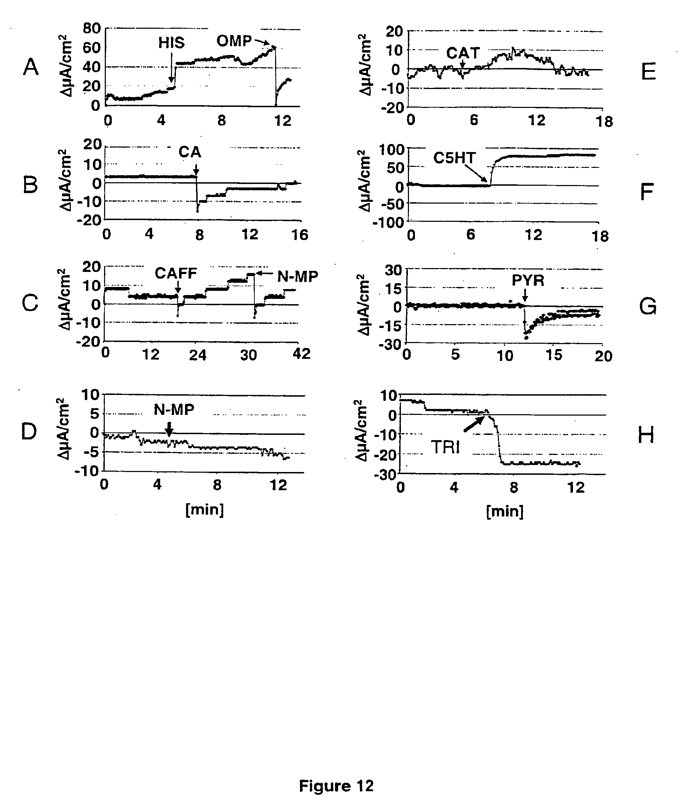

Figure 12: Measurement of secretory activity in the Ussing chamber. A: Treatment with histamine (HIS) increased the current after apical admission. The increased current was subsequently decreased by apical admission of omeprazol (OMP), a specific inhibitor of the H+, K+-ATPase. B: Apical admission of chlorogenic acid (CA) caused a decreased current indicating a decreased secretory activity as in (A). C: Apical admission of caffeine (CAFF) increased the current. After basal admission of N-methylpyridinium (N-MP) the current decreased again indicating an inhibitory effect of N-MP. D: basal admission of N-MP alone only caused a slightly decreased current. E:. Apical addition of catechol (CAT) increased the current, but the peak maximum was not reached at point of admission indication slow response of parietal cells to CAT. F: Apical addition of C5HT strongly increased the current at point of admission, however, the IPX did not turn out for significance (p=0.07, see A). G: Pyrogallol (PYR) caused a drop of current after apical admission indicating a decreased secretion. H: Basal addition of trigonelline (TRI) caused a strong decrease in current, indicating an anti-secretory potential.

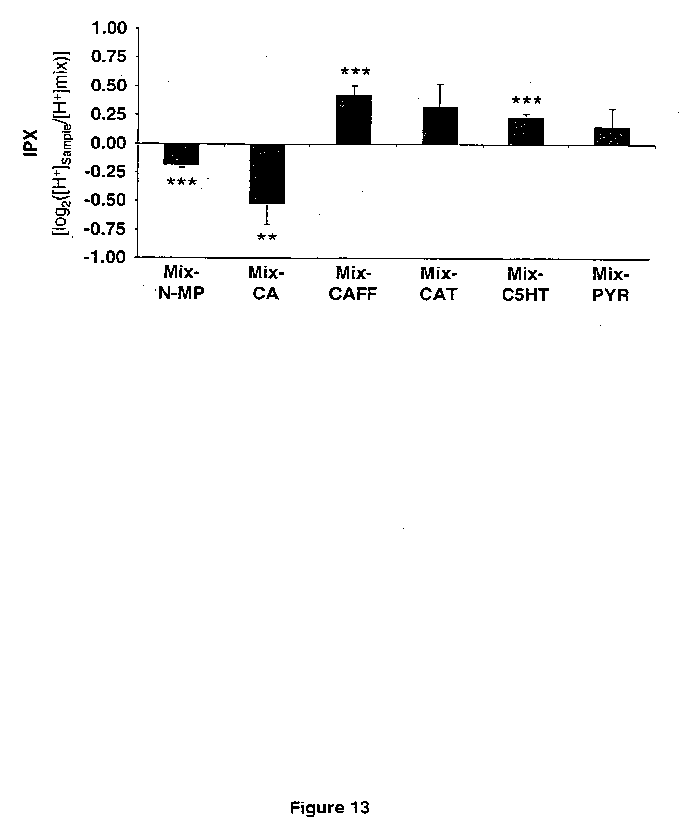

Figure 13: Recombinative effects of tested compounds. Either N-methylpyridinium (N-MP), chlorogenic acid (CA), caffeine (CAFF), catechol (CAT), C5HT or pyrogallol (PYR) were omitted from the full recombination of all tested compounds (Mix) to elucidate synergistic effects of each compound. (Statistics: one-tailed or two-tailed t-test for mix without compound vs. mix, **=p=0.01, ***=p=0.001, n=9)

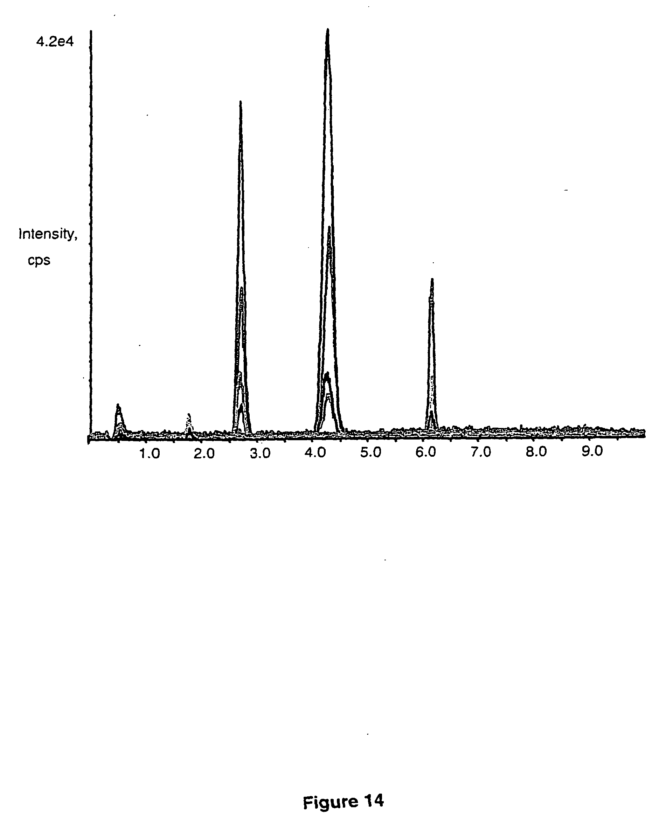

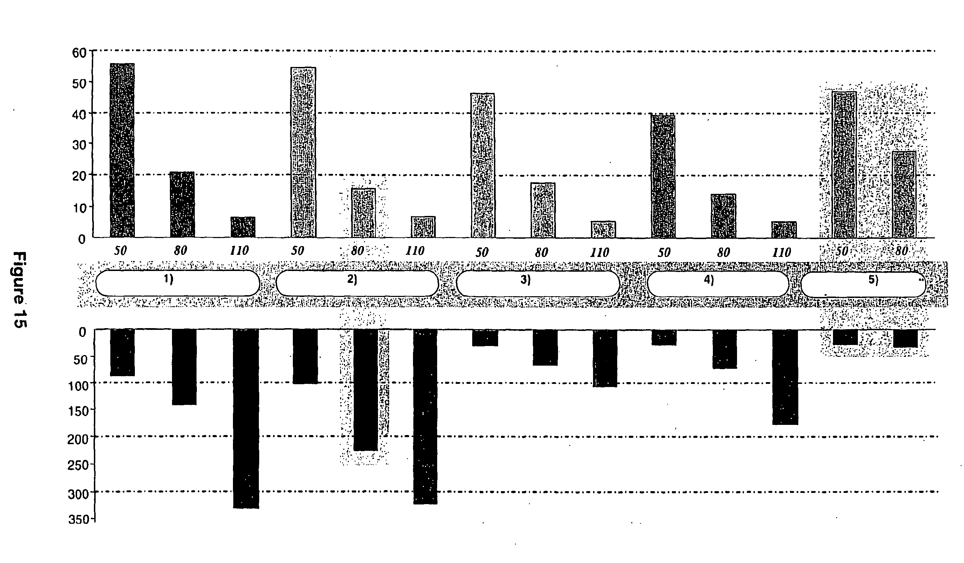

Figure 14: HPLC-MS/MS (ESI⁺) analysis of a roast coffee filter drinks. Besides the C5HT, N-methylpyridinium was determined in the same drink using a stable isotope dissolution analysis, which was already successfully employed in previous studies. The data are summarized in Fig. 15.
Figure 15: Summary of the determined data concerning N-alkanoyl-5-hydroxytryptamides (lower panel, µg/l, sum of the derivatives) and N-methylpyridinium (upper panel, mg/l). 50, 80, 110 denote the roast degree in scale parts. 1) "untreated Col 5", 2) "untreated Col 2", 3) "decaffeinated 2+2", 4) "dewaxed Col 5", "dewaxed Col 2**"

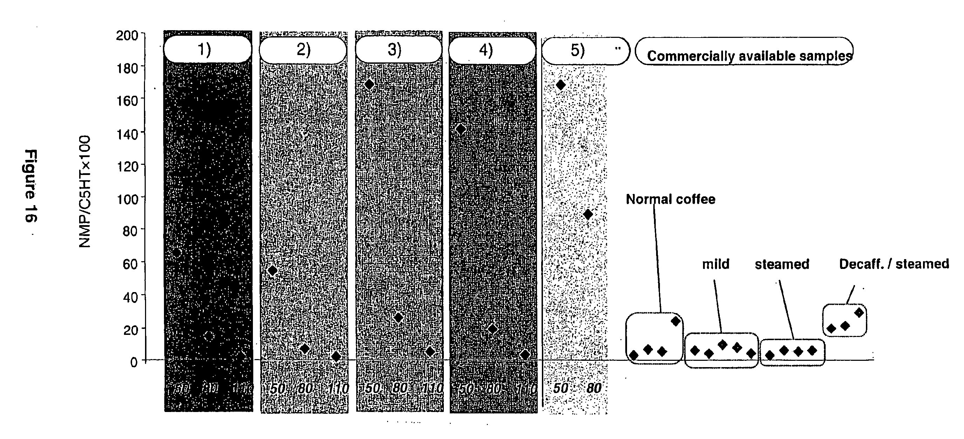

Figure 16: Comparison of the analyzed coffees on the basis of the NMP/C5HT-relation (factor = NMP/C5HTx100). 50, 80, 110 denote the roast degree in scale parts. Coffees of previous studies are shown for comparative reasons. 1) "untreated Col 5", 2) "untreated Col 2", 3) "decaffeinated 2+2", 4) "dewaxed Col 5", 5) "dewaxed Col 2**"

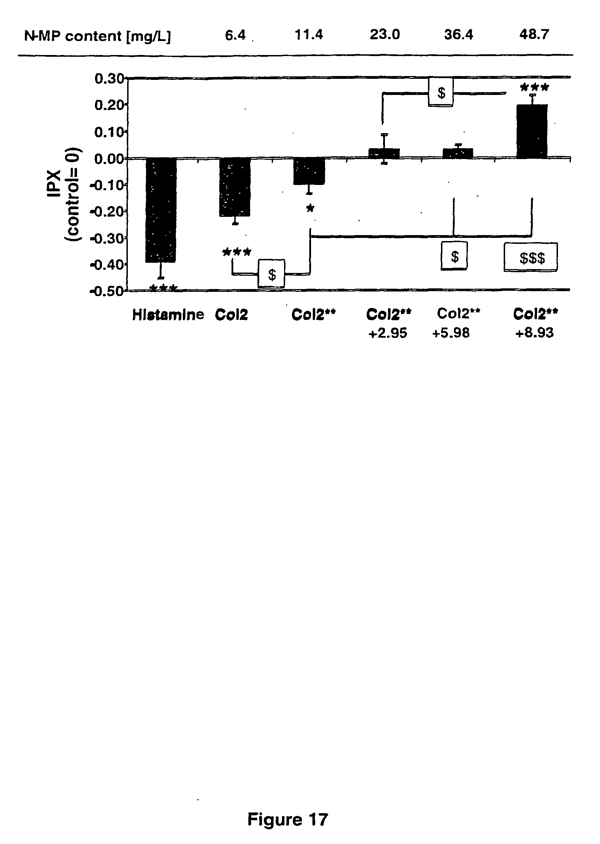

Figure 17: Comparison of the secretory potential of coffees enriched with N-MP The intracellular proton index (IPX) is shown. The most effective N-methylpyridinium concentrations (in the resuspended lyophilisate) are shown above the figure (statistics: two-tailed t test, ***=ps0.001, $ = p<0.05, $$ = p<0.01; $$$ = p<0.001).
Figure 18: Influence of trigonelline on the secretory activity (statistics: two-tailed t-test, ***=p≤0.001).
Figure 19: Dose-effect-relation of trigonelline (statistics: one-way ANOVA; *=p≤0.05, $ = p<0.05, $$ = p<0.01, $$$ = p<0.001, n=4)
Figure 20: Influence of trigonelline and N-methylpyridinium on the short circuit current.

The present invention will now be further illustrated in the following examples without being limited thereto.

### Examples

### Example 1: Activity-Guided Fractionation to Characterize a Coffee Beverage that Effectively Down-Regulates Mechanisms of Gastric Acid Secretion As Compared to Regular Coffee

In some individuals, consumption of coffee beverages is often related to symptoms of gastric irritation. Hot water steam-treatment of the raw coffee bean is hypothesized to substantially reduce the contents of stomach irritating compounds and products to which this technology is applied are launched as 'stomach-friendly coffee'. However, data on the effect of steam-treated coffee on gastric acid secretion are conflicting and it has not been proven yet which coffee components act as pro- or anti-secretory stimulants. The here presented example aimed at the characterization of a coffee beverage that effectively down-regulates mechanisms of proton secretion in human gastric cells (HGT-1).

At first, a regular coffee beverage was fractionated by using solvents of different polarity: water, ethylacetate, dichloromethane, and pentane. Functional assays on the proton secretory activity (PSA) of these solvent fractions revealed the least pronounced effect for the water fraction, for which quantitative analyses demonstrated the highest distribution of chlorogenic acid (95 %), βN-alkanoyl-5-hydroxytryptamides (55 %) and N-methylpyridinium (N-MP, >99%) among all fractions. Following experiments demonstrated that HGT-1 cells treated with regular coffee fortified with N-MP at a concentration of about 20 mg/mL N-MP showed a significantly decreased PSA as compared to cells which were exposed to coffee beverage lyophilizates containing higher (32-34 mg/L) or lower (5 mg/L) N-MP concentrations. These results were proven by applying pathway analyses of transcription (ATF-1, Akt1) and signaling (cAMP, EGFr) factors, kinases (ERK1/2) and by experiments on the gene expression of pro- (histamine-HRH2, acetylcholine-CHRM3) and anti- (somatostatin-SSTR1) secretory receptors, and the H+,K+-ATPase.

***Sample Preparation***. For solvent fractionation, ground roast coffee powder (regular commercial coffee blend neither steam-treated nor de-caffeinated), 54 g of homogeneous material were weighed into a paper filter commonly used for home preparation of coffee beverage (Melitta Gold, Nr. 4, Aldi, Germany). Portions of freshly boiled tap water (T-90-95 °C, approximately 100 mL each) were poured over the powder and the hot filtrate was collected in a volumetric flask of 1000 mL. Exactly 500 mL of the coffee solution was transferred to crystallizing dishes, frozen (-20 °C) immediately, and finally dried by lyophilization (48 h, 0.77 mbar, 25°C). The second part of the coffee beverage (500 mL) was transferred to a separation funnel, and the aqueous phase was sequentially extracted with pentane (4 x 500 mL), dichloromethane (4 x 500 mL), and ethyl acetate (4 x 500 mL), with aqueous phase as residue. All fractions obtained were reduced in a vacuum, taken up in water, frozen (-20°C) and freeze-dried (48 h, 0.77 mbar, 25 °C). The yields were determined by gravimetry. Solvent fractions were then used for functional assays on the secretory activity of human gastric parietal cells in concentrations according to their respective yields.

Studies on the cellular mechanisms of gastric acid regulation were performed with two *C. Arabica* Brazil (coffee A) and two *C. robusta* Vietnam (coffee R) samples. One of each coffee varieties was a regular coffee, neither steam-treated nor de-caffeinated, the other matching sample was subjected to steam-treatment (coffee AT, coffee RT), based on the manufacturer's practice to remove putative stomach irritating. All coffee samples were representative for commercial coffees and were provided by a local coffee manufacturer. Briefly, raw coffee beans are subjected to hot water-steam extraction at a pressure of 19 to 22 psi for 10 - 30 minutes. Afterwards, the remaining humidity is evaporated by heating the beans up to 130 - 150 °C. Finally, the evaporated water steam is released from the apparatus, whereas the original water content of raw coffee beans is restored in a vacuum atmosphere. From each of these four coffees (A, AT, R and RT), beverages were prepared following a standard recipe which is typically used for household preparations.

***Cell culture.*** Human parietal carcinoma cells (HGT-1) were provided by Dr. C. Laboisse (Laboratory of Pathological Anatomy, Nantes France) and cultured at 37°C and 5% CO2. Dulbeccos Modified Eagle Medium (DMEM, PAA, Coelbe, Germany) with glucose (4%, PAA, Coelbe, Germany) was used as culture medium and supplemented with 20% fetal calf serum (PAA, Coelbe, Germany), 2% L-glutamine (PAA, Coelbe, Germany), 2% penicillin streptomycin (PAA, Coelbe, Germany) and 2% 4-(2-hydroxyethyl)-1-piperazineethane-sulphonic acid (HEPES)-buffer (PAA, Coelbe, Germany). Prior to each of the experiments, cells were cultivated for 5 days and synchronized with DMEM that was not supplemented with fetal calf serum.

***RNA isolation and cDNA synthesis.*** 100,000 cells were seeded in six-well plates. After sample treatment, cells were harvested for the analysis of total RNA which was isolated using the RNeasy Midi Kit (Qiagen, Hilden, Germany). DNase I digest was performed on-column with RNase free DNase Kit (Qiagen, Hilden, Germany). Prior to qPCR, total RNA contents were quantitated photometrically at 260 nm. The cDNA was synthesized using the cDNA High Capacity Synthesis Kit (Applied Biosystems, Munich, Germany), as described in the manufacturer's manual.

***Gene Expression Assays.*** Gene expression analyses was performed after sample treatment of the cells for 5, 10, 15 or 20 min. Primers for the H+,K+-ATPase alpha-subunit (*ATP4A*), the histamine H2 receptor (*HRH2*), the somatostatin receptor (*SSTR2*) and the acetylcholine receptor M3 *(CHRM3*) were designed with Beacon Designers 7.0 (PremierBiosoft, Palo Alto, CA), and validated by standard and melting curve analysis as stated elsewhere. The correct sequence of PCR products was verified by sequencing (Medigenomics, Martinsried, Germany, data not shown). Realtime-PCR assays were performed on a Mx3000p cycler (Stratagene, Amsterdam, Netherlands) using the Brilliant SYBR Green Kit (Stratagene, Amsterdam, Netherlands). Cycling conditions were as follows: 10:00 min /95°C (activation), 00:30 sec/95°C (denaturation), 00:30 sec/60°C (annealing with fluorescence measurement), 00:30 sec/72°C (elongation).

***Quantitative determination of cyclic AMP.*** A total of 50,000 cells were seeded in 24-well plates and treated with the respective sample for 0.5 minutes. For the determination of cyclic AMP in cell supernatants, we applied the competitive cyclic AMP ELISA parameter kit (R&D Systems, Minneapolis, MN) as described in the manufacturer's protocol. Supernatants were isolated after 1 minute of incubation.

***Signal transduction assay.*** Phosphorylation status of receptor tyrosine kinases and MAPK kinases was detected by enzyme linked immunoassays. Total protein was isolated from HGT-1 cells after incubation with the tested coffees for 10 min as recommended by the manufacturer's protocol. Total protein content was quantified photometrically using Bradford's reagent (Bio-Rad, Munich). The following ELISAs were used for determination of the phosphorylation status: EGFr-ELlSA Kit, ERK1/2 ELISA Kit (both Calbiochem/Merck, Nottingham, UK), Akt1 pathscan ELISA and ATF-2 pathscan ELISA (both Cell Signaling/New England Biolabs, Frankfurt a. M., Germany). Absorption was read out at 450 nm on a MRX plate reader (Dynex, Berlin, Germany) or Varioscan Flash plate reader (Thermo Electron Cooperation, Walthman, MI) with additional reading of the reference wave length at 620 nm if indicated by the manufacturer's protocol.

***Secretory Activity**.* A volume of 2 mL cell suspension, corresponding to 2,000,000 cells, was used for each biological independent experiment and was treated for 10 minutes with 2.5 mg coffee beverage lyophililsate (54 g / 1.1 L hot water) per mL PBS, histamine (1 mmol in PBS) or different solvent fractions at 37°C. The secretory activity was measured by the determination of the intracellular proton index (IPX). The fluorescence dye Carboxy-SNARF-AM (Invitrogen, Karlsruhe, Germany) was used for the measurement of the intracellular pH. The method was validated as described elsewhere and histamine (1 mmol/L) was used as a well-known reference compound that stimulates stomach acid secretion. Briefly, HGT-1 cells were loaded with 3 µM dye for 30 minutes on-ice. The intracellular pH was calculated referring to a calibration curve with 2 µM nigericin (PAA, Coelbe, Germany) treated HGT-1 cells in K+ clamp buffer consisting of 20 mM NaCl, 110 mM KCI, 1 mM CaCl2, 1 mM MgSO4, 18 mM D-Glucose and 20 mM HEPES that was set to different pH calibration points (6.8-8.2) by titration with NaOH. Then, the intracellular proton index (IPX) was calculated by log2 transformation of the intracellular proton concentration ratio between treated cells and control cells. The higher the IPX, the more protons remain in the cell, indicating a lower secretory activity, whereas lower IPX values indicate a higher proton secretion.

***Statistical analysis.*** Statistical analysis was performed with Excel 2003 or SigmaStat (Systat Software GmbH, Erkrath, Germany). Data sets generated by qPCR and ELISA were transformed by logarithmic conversion to reach normal distribution and are displayed as indexed log2 ratio in the figures. Single comparisons between treated and control cells were done with the two-tailed Student's t-test for equal variances. For time course analysis of gene expression, we performed the one-way ANOVA with Holm-Sidak post-hoc analysis for parametric data sets and the Kruskal-Wallis test with Dunn's post-hoc analysis for non-parametric data sets. Numbers of replicates for each experiment are stated in the results part, but were at least n=3. In each diagram, the error bars represent the standard error (SE).

***Secretory activity of solvent fractions prepared from regular coffee.*** The following approach to evaluate the gastric acid secretory activity of coffee beverages in human gastric cancer cells (HGT-1) was used. Cells treated with lyophilized coffee beverages prepared from de-caffeinated and/or steam-treated coffee showed a reduced secretory activity compared to cells treated with lyophilizates prepared from regular coffee. In this model, the intracellular pH as indicator of proton secretion is analyzed by flow cytometry using the pH-sensitive dye SNARF-AM. Data are calculated as intracellular pH index (IPX), based on the hypothesis that the intracellular concentration of H+ decreases due to proton secretion. The higher the IPX, the more protons remain in the cell, indicating a lower secretory activity, whereas lower IPX values indicate a higher proton secretion. IPX values are finally log2 transformed for variance stabilization. In the work presented here, treatment of HGT-1 cells with the physiological stimulant histamine lead to a stimulation of secretory activity as indicated by a significant decrease of the I PX (-0.41 ±0.04; p.0.001) compared to non-treated control cells (Fig. 1). Cells treated with those fractions extracted from a commercial regular coffee blend with ethyl acetate, dichloromethane and pentane reacted in a similar way with IPX values of -0.43±0.6, -0.20±0.03 and - 0.34±0.04, respectively (two-tailed t-test, p.0.001 vs. non-treated control cells for each fraction; Fig. 1). In contrast, cells exposed to the water extract demonstrated a significantly decreased secretory activity compared to control cells, as indicated by positive IPX values of +0.07±0.03 (two-tailed t-test, p.0.05; Fig. 1). Considering the distribution of the quantitated compounds among the solvent fractions, the pentane fraction contained only one of them: Caffeine, with about 1 % of the total amount quantitated in all fractions.

Surprisingly, most of the compounds quantitated were distributed among the water, the ethyl acetate and the dichloromethane fraction, with catechol and pyrogallol predominantly extracted with ethyl acetate and caffeine mainly ending up in the dichloromethane extract. However, the water extract, which showed anti-secretory activity in the functional assay, contained the majority of chlorogenic acid and βN-alkanoyl-5-hydroxytryptamides, and was the only extract that contained more than 99 % of the total amount of N-methylpyridinium quantitated (Fig. 1).

***Secretory activity of regular coffee lyophilizates after addition of N-methylpyridinium.*** Since results from the experiments with solvent fractions isolated from the commercial regular coffee blend suggested an anti-secretory effect of coffee rich in water soluble compounds, and, in particular N-methylpyridinium, which was solely quantitated in the water extract, N-methylpyridinium as single compound as well as a lyophilizate prepared from a regular coffee to which N-methylpyridinium was added were studied for their secretory activity in HGT-1 cells. Quantitation of N-methylpyridinium in coffee A revealed a concentration of 6.32 mg/L (Fig. 2). Fortification of the beverage lyophilizate was performed by adding different amounts to achieve a 2-fold (12.64 mg/L), a 3-fold (18.96 mg/L), a 5-fold (31.60 mg/L) and a 10-fold (63.20 mg/L) increase compared to the non-fortified lyophilizate. As shown in Fig. 2, treatment of the HGT-1 cells with the non-fortified coffee lyophilizate had a stimulatory effect on the secretory activity, with an IPX of -0.17±0.03 (two-tailed t-test, p.0.001 vs. non-treated control cells), whereas in cells treated with lyophilizates with two and three times more N-methylpyridinium, significantly lower secretory activity was analyzed (-0.10±0.04 and -3.46-17±0.01, respectively, two-tailed t-test, p.0.01 vs. non-treated control cells for both samples). The lyophilizate containing 3-fold higher N-methylpyridinium concentrations compared to the non-fortified lyophilizate in fact did not seem to have an effect at all, as cells treated with this samples showed the same IPX as non-treated control cells. However, with increasing amounts of N-methylpyridinium up to 10-fold (63.2 mg/L), the secretory activity of coffee A is almost the same as was analyzed for the non-fortified lyophilizate (one-way ANOVA, p.0.05; Fig. 2).

When HGT-1 cells were treated with N-methylpyridinium as a single compounds, no dose dependent effect was observed (Fig. 2). These results clearly demonstrate that N-methylpyridinium does act as an anti-secretory compound in coffee beverage, but not as a single substance.

***Secretory activity of lyophilizates prepared from standardized coffee beverages with varying contents of N-methylpyridinium and their effect on cyclic AMP formation.*** Since the experiments with N-methylpyridinium fortified lyophilizates prepared from coffee A suggested an anti-secretory effect of coffee beverages having a 3-fold higher N-methylpyridinium concentration as compared to non-fortified lyophilizates, three additional coffee samples with defined N-methylpyridinium contents were produced. At first, a matching sample to coffee A consisted of the same coffee, but was mildly steam-treated (coffee AT) in such a way that the N-methylpyridinium content was not affected (Table 1). For the second set of samples, a *C. robusta* Vietnam (coffee R) and a *C. robusta* Vietnam - steam-treated (coffee RT) were produced. Quantitation of N-methylpyridinium in these two samples revealed a 'medium high' and a `low' concentration, with 22.4 mg/L and 5.41 mg/l, respectively (Table 1). Thus, coffee R contained comparable N-methylpyridinium concentrations as coffee A fortified with up to 18.96 mg/L.

IPX analyzed in HGT-1 cells after treatment with the lyophilizates prepared from coffee A (-0.43±0.05). AT (-0.49±0.02) and R (-0.35±0.07) demonstrate a significantly lower secretory activity compared to coffee RT (-0.60±0.02) (Fig. 3A). Thus, the 'high' and 'medium high' concentrations of N-methylpyridinium quantitated in coffee A / AT and R, respectively, seem to be associated with a lower secretory potential compared to coffee RT with the lowest N-methylpyridinium concentrations quantitated in those for coffee samples.

Stimulation of gastric acid secretion is related to elevated cyclic AMP levels in parietal cells since gastrin and histamine receptor signaling increase the synthesis of cyclic AMP by adenylatcyclase due to G-protein activation. In this study, HGT-1 cells treated with histamine stimulated the secretory activity significantly to an IPX of -0.97±0.02 compared to non-treated control cells. In accordance with this result, also the cyclic AMP concentration was increased to 7.76±0.69 pmol/mL from a base level of 6.02±0,36 pmo/mL in non-treated control cells (two-tailed t-test, p≤0.05; Fig. 3A and B). In accordance with the results from the secretory activity assay, the cyclic AMP concentration after HGT-1's treatment with coffee RT (19.69±0,41 pmol/mL) was significantly higher (two-tailed t-test, p≤0.05) compared to control cells and to the cyclic AMP concentrations after treatment with coffee A (8.83±0,69 pmo/mL), coffee AT (10.76±0.38 pmol/mL) or coffee R (14.98±0.89 pmol/mL) (Fig. 3B). Overall, coffee RT stimulated the proton secretion compared to control cells more than any other coffee lyophilizate tested, whereas resulted in the highest intracellular cyclic AMP levels. Coffee R, in contrast, exhibited the least stimulatory effect on the proton secretory activity, but induced the second highest cyclic AMP levels in the HGT-1 cells. These results suggested that the coffee lyophilizates tested activate gastric secretion by more pathways than cyclic AMP signaling.

***Effect of coffee lyophilizates on signal transduction pathways and on gene expression.*** Signal transduction bridges the gap between receptor activation and regulation of gene transcription of functional proteins, e. g. H+,K+-ATPase. So far EGFr, ERK1/2 and Akt1 signaling have been shown to be involved in gastric acid secretion. The regulation of gene transcription depends on transcription factors. Since the H+,K+-ATPase and the somatostatin receptor hold binding motifs for cyclic-AMP dependent transcriptions factors, we examined also the role of ATF-2 in the process of gene regulation and secretory activity. As a result, exposure of HGT-1 cells to histamine resulted in ATF-2 activation (pathway activation index 1.17±0.25 vs. 0 for non-treated control cells; p≤0.001, Fig. 4). For all of the coffee lyophilizates tested, an inductive effect of ATF-2 activation was demonstrated which was lowest for coffee R (coffee R: 0.33±0.13; coffee RT: 0.84±0.39; coffee A: 0.97±0.15; coffee AT: 0.85±0.20; each p-0.05 vs. non-treated control cells; Fig. 4). However, coffee RT did not affect the expression of the pathway related gene of the H+,K+-ATPase alpha-subunit (ATP4A), whereas cells treated with coffee R showed an enhanced gene expression of the anti-secretory somatostatin receptor (0.74±0.19, one-way ANOVA p≤0.01 vs. non-treated control cells (=0), Fig. 5A and B). When steam-treated coffee R (coffee RT) was studied, no effect was demonstrated for the somatostatin receptor, but also an increase in ATP4A gene expression was demonstrated (0.55±0.04, one-way ANOVA p≤0.05 vs. non-treated control cells (=0), Fig. 5A and B). HGT-1 cells treated with coffee A showed an increase in ATP4A gene expression (0.47±0.09, p≤0.05 vs. non-treated control cells (=0)), whereas the gene expression of the anti-secretory somatostatin receptor was decreased (-0.59±0.17, one-way ANOVA p≤0.01 vs. non-treated control cells (=0), Fig. 6A and B).

Another transcription factor that is reported to enhance gastric acid secretion and the gene expression of the H+,K+-ATPase is Akt1. In this study, Akt1 signaling was activated significantly in HGT-1 cells exposed to coffee AT, R or RT with maximum pathway activation indices of 1.75±0.34, 0.60±0.22 and 0.75±0.30, respectively (two-tailed t-test, p≤0.05-0.01 vs. non-treated control cells; Fig. 4). However, neither coffee AT nor coffee R affected the gene expression of ATP4A. Treatment of the HGT-1 cells with coffee A, in contrast, caused a slight, but significant decrease of Akt1 activation compared to non-treated control cells (-0.22±0.05, two-tailed t-test p≤0.05, Fig. 6). Since coffee A increased ATP4A expression, Akt1 signaling appeared not to be the triggering factor for ATP4A expression after exposure to coffee. Nevertheless, when HGT-1 cells were exposed to coffee A, ERK1/2 was activated (1.30±0.58, two-tailed *t*-test vs. non-treated controls p≤0.05, Fig. 4). In contrast, ERK1/2 signaling was decreased after treatment with coffee RT (-0.58±0.49, two-tailed *t*-test, Fig. 4).

EGFr receptor activation was significant after HGT-1 exposure to histamine (1.32±0.42) and coffee RT (0.58±0.23), solely (two-tailed t-test, p≤0.05-0.001, Fig. 4). Here, again a higher secretory activity of coffee RT is indicated. Considering the expression of pro-secretory receptors, the histamine H2 receptor was not impaired by incubation of HGT-1 cells with coffee A or AT. Coffee RT, in contrast, increased its expression significantly (0.41 ±0.10, one-way ANOVA *p*≤0.01 vs. non-treated control cells, Fig. 5C). The pro-secretory acetylcholine receptor M3 was decreased in its gene expression after treatment of the HGT-1 cells with coffee A (-0.56±0.09, one-way ANOVA p≤0.001, Fig. 6B). Coffee R and RT, in contrast, showed an enhancing effect on the gene expression of the acetylcholine receptor M3 (0.43±0.12 and 0.71±0.23, respectively, one-way ANOVA, p≤0.01 vs. non-treated control cells, Fig. 6B). Taken together, the treatment of parietal cells with coffee RT resulted in the highest secretory activity, most comprehensive up-regulation of pro-secretory genes and activation of gastric acid secretion related pathways. HGT-1 cells exposed to coffee AT did not show any increase in the expression of pro-secretory genes nor could a decrease in the expression of anti-secretory genes be observed. In line with that, the secretory activity of parietal cells after exposure to coffee AT was below coffee RT (Fig. 3).

The least pronounced effect on the secretory activity was demonstrated for coffee R (Fig. 3) which did show an increasing effect on the gene expression of the pro-secretory acetylcholine receptor M3 but also demonstrated the highest stimulating effect on the gene expression of the anti-secretory somatostatin receptor among all coffees tested (Fig. 5B).

**Table 1: Concentrations of major putative bioactive compounds in coffee beverages (mg/L) and in lyophilizates (mg/L) prepared from either c. Arabica Brazil (Coffee A), c. Arabica Brazil steam-treated (Coffee AT), c. Robusta Vietnam (coffee R) or c. Robusta Vietnam steam-treated (coffee RT). In addition, the ratios of the single compounds between the coffee lyophilizates are given, where coffee with the lowest content of a certain compound was set to 100%.**

| **Coffee beverage [mg/L]** | **Coffee A** | **Coffee AT** | **Coffee R** | **Coffee RT** |
|---|---|---|---|---|
| Extraction yield^{a} | 12740 | 12580 | 15800 | 16750 |
| Chlorogenic acid^{b} | 1038 | 1083 | 975 | 1464 |
| Caffeine^{b} | 618 | 594 | 1389 | 1326 |
| N-methylpyridinium^{b} | 32.21 | 34.70 | 22.40 | 5.41 |
| Pyrogallol^{b} | 4.05 | 3.99 | 5.63 | 4.79 |
| Catechol^{b} | 5.73 | 5:29 | 9.26 | 4.43 |
| C5HT^{b} | 0.21 | 0.21 | 0.12 | 0.08 |
| | | | | |
| **Effective dosage in the lyophilisate [mg/L]** | | | | |
| Chlorogenic acid^{c} | 203.66 | 215.22 | 154.27 | 218.51 |
| Caffeine^{c} | 121.27 | 118.04 | 219.78 | 197.91 |
| N-methylpyridinium^{c} | 6.32 | 6.90 | 3.54 | 0.81 |
| Pyrogalloi^{c} | 0.79 | 0.79 | 0.89 | 0.71 |
| Catechol^{c} | 1.12 | 1.05 | 1.47 | 0.66 |
| C5HT^{c} | 0.04 | 0.04 | 0.02 | 0.01 |
| | | | | |
| **Ratios** | | | | |
| Chlorogenic acid | 132% | 140% | 100% | 142% |
| Caffeine | 103% | 100% | 186% | 167% |
| N-methylpyridinium | 780% | 851% | 437% | 100% |
| Pyrogallol | 111% | 111% | 125% | 100% |
| Catechol | 170% | 159% | 221% | 100% |
| C5HT | 400% | 400% | 200% | 100% |

| | | | | |
|---|---|---|---|---|
| ^{a} extraction yield determined by weight after lyophilisation ^{b} concentration determined by HPLC-DAD/HPLC-MS/MS techniques in the freshly prepared beverage ^{c} concentrations calculated from concentrations in the beverage and the extraction yields. **Coffee A:** Arabica Brazil untreated; **Coffee AT:** Arabica Brazil, steam treated; **Coffee R:** Vietnam Robusta untreated; **Coffee RT:** Vietnam Robusta, steam treated | | | | |

The above example aimed at the compositional characterization of a coffee beverage that effectively down-regulates selected mechanisms of gastric acid secretion. First, a regular coffee beverage, neither steam-treated nor decaffeinated, was subjected to solvent fractionation in order to identify the polarity of compounds that affect the proton secretary activity in HGT-1 cells. In addition, each of these solvent fractions obtained from the extraction of the coffee beverage with water, ethyl acetate, dichloromethane or pentane was analyzed for its contents of putative stomach irritating compounds, namely caffeine, chlorogenic acid, βN-alkanoyl-5-hydroxytryptamides, pyrogallol, catechol as well as N-methylpyridinium as another recently identified bioactive compound in coffee beverage. The functional assays revealed a stimulative effect on the proton secretory activity for the ethyl acetate, the dichloromethane and the pentane extract, whereas no effect on the proton secretion was demonstrated for the water extract. This result was somewhat unexpected, as the water extract contained the majority of chlorogenic acid (95 %) and βN-alkanoyl-5-hydroxytryptamides (55 %) present in the total coffee beverage. Both compounds are hypothesized to promote stomach acid secretion. However, we attribute the absence of a stimulating effect on proton secretion in HGT-1 cells to the content of N-methylpyridinium in the water fraction which solely contained this compound. Although we could not prove this effect in experiments in which HGT-1 cells were treated with N-methylpyridinium as a single compound, addition of N-methylpyridinium to a lyophilizate prepared from a regular coffee beverage (neither steam-treated nor de-caffeinated) clearly demonstrated an anti-secretory effect for this compound at a concentration of about 18 mg/l beverage. These differences between the single compound and the complex beverage seem reasonable, since a coffee beverage contains a variety of components that may act as competitors for receptor binding, act as inhibitors or enhancers of functional proteins as well as for gene regulation and finally may exhibit synergistic or anti-synergistic effects.

To further evaluate the effect of coffee beverages with varying concentrations of N-methylpyridinium on cellular mechanisms of stomach acid secretion, lyophilizates prepared from coffee beverages with *high* (C. Arabica: 32 mg/L and C. Arabica Brazil mildly steam-treated: 34 mg/L), *medium* (C. Robusta Vietnam: 22 mg/L) and *low* (C. Robusta Vietnam steam-treated: 5 mg/L) concentrations were obtained from a local coffee manufacturer. Steam-treatment was applied according to the procedure used for the production of commercial 'stomach-friendly'-labeled coffees. The results from the functional experiments investigating the effect of the lyophilized coffee beverage on the proton secretory activity revealed a significantly lower secretory activity for those coffee beverages with medium or high concentrations of N-methylpyridinium as compared to the beverage with the lowest concentration. However, the least secretory activity was demonstrated for C. Robusta Vietnam, with a N-methylpyridinium concentration of 22 mg/l beverage. This result is in accordance with the findings obtained from experiments in which a lyophilizate prepared form a commercial regular coffee was fortified with N-methylpyridinium up to a final concentration of 18 mg/L beverage. Also, results on the molecular mechanisms of stomach acid secretion, such as gene regulation, signal transduction pathways and related transcription factors demonstrate the least pro-secretory effect among the coffee beverages studied for C. Robusta Vietnam. In particular, an inducing effect was shown on the gene expression of the anti-secretory receptor somatostatin (SSTR2), whereas no effect on the gene expression of the H+,K+-ATPase was observed.

Surprisingly, the least secretory activity among the coffee samples tested was correlated with an increased gene expression of the acetylcholine receptor (CHRM3). This result indicates that the pathways regulating the stomach acid secretion differ in their effectiveness. Up-regulation of pro-secretory genes or transcription factors protein does not necessarily result in an increased proton secretion and *vice versa.* This is also demonstrated by the results for the other coffee beverages studied in this work. The steam-treated C. Robusta Vietnam, for example, showed the most stimulating effect on the proton secretory activity, an up-regulative effect on the gene expression of the H+,K+-ATPase, the key player of gastric acid secretion, and also on the gene expression of the pro-secretory histamine (HRH2) and acetylcholine receptor (CHRM3). In contrast to these pro-secretory effects, HGT-1 cells treated with C. Robusta Vietnam also showed decreased concentrations of cyclic AMP and decreased activation of ERK1/2, both involved in pro-secretory pathways.

In conclusion, we have shown for the first time that regulation and function of the proton secretory activity of human stomach cells induced by coffee beverages depend on their concentrations of N-methylpyridinium. The impact of other components such as caffeine, chlorogenic acid or βN-alkanoyl-5-hydroxytryptamides are examined in the following multi-parametric studies.

### Example 2: Multi-parametric approach to identify coffee components that regulate mechanisms of gastric acid secretion

In this study, six different compounds, chlorogenic acid (CA), caffeine (CAFF), pyrogallol (PYR), catechol (CAT), N-alkanoyl-hydroxytryptamides (C5HT) and N-methylpyridinium (N-MP) were evaluated for their influence on secretory activity, signal transduction and expression of gastric acid related genes as well as the activation of the transcription factor ATF-2 as well as cAMP concentrations. We elucidated which compounds may act as a stomach protective compound in coffee beverages. Furthermore, we developed a statistical modeling to predict synergistic and anti-synergistic effects of multi-compound solutions on gastric secretion.

N-MP, CAFF, CAT, C5HT, PYR and CA were used in concentrations according to a regular coffee beverage. The human gastric cancer cell line HGT-1 was used for the *in vitro* studies. Secretory activity was measured by Ussing chambers and FACS analysis. To detect activation of EGFr, Akt1, ERK1/2, ATF-1 and cAMP levels we performed ELISA assays whereas changes in time dependent expression of gastric acid related genes like the histamine, acetylcholine and somatostatin receptors as well as the H+, K+-ATPase were determined by real-time PCR. The experimental data was then partially used for the statistical modeling using the program "neuronal networks".

N-MP increased the expression of the anti-secretory somatostatin receptor by 114% whereas C5HT decreased its expression to 48%. Furthermore, N-MP down-regulated the pro-secretory acetylcholine receptor M3 (-36%) and the H+, K+-ATPase by -27%. The ATPase was 2-fold up-regulated by CA and CAFF. CAFF most strongly stimulated the secretory activity in the functional assays. N-MP acted as inhibitor of secretory activity induced by CAFF. Surprisingly, CA did not increase secretion than rather lead to a decrease in secretion. Pathway analysis was able to discriminate between CAFF, CA, CAT, C5HT, PYR and histamine activating EGFr signaling and N-MP activating Akt1 and ERK1/2 signaling. In summary, these results suggest a stomach protective potential for pyrogallol and N-methylpyridinium *in vitro.*

***Cell culture**.* HGT-1 (Dr. C. Laboisse, Nantes, France) cells were cultured at 37°C and with 5% CO2 in Dulbecco's modified Eagle medium with 20% fetal calf serum, 5% glutamine and 5% penicilline/streptomycine. The cells were synchronized for 24 hours prior to experiments with Dulbecco's modified Eagle Medium as described above, but without containing fetal calf serum.

***Single compound experiments**.* Cells were exposed to caffeine (3 mM), chlorogenic acid (3 mM), catechol (52 µM), pyrogallol (32 µM) (all Sigma-Aldrich, Munich, Germany), βN-alkanoyl-5-hydroxytryptamides (1 µM) and N-methyl-pyridinium (0.34 µM), in concentrations according to their contents quantified in a regular coffee beverage. The other concentrations were omeprazole (1 mM), somatostatin (0.5 mM), histamine (1 mM), acetylcholine (1 mM) (all Sigma-Aldrich, Munich, Germany). All compound were either directly resolved in phosphate buffered saline (PBS) or in Ringer's solution, except for N-alkanoyl-5-hydroxytryptamides that where resolved in tetrahydrofuran (1mg/mL THF) and was added in a volume of 2 µl to 10 ml of buffer in order to reach the final concentration.

***Expression Assays**.* After 5, 10, 15 and 20 min of exposure, cells were harvested for the analysis of gene expression. Primers for *ATP4A, HRH2, SSTR2* and *CHRM3* were designed with Beacon Designer 7.0 (PremierBiosoft, Palo Alto, CA) and validated by standard and melting curve analysis. The correct sequence of PCR products was verified by sequencing (Medigenomics, Martinsried, Germany). Realtime PCR assays were performed using the Brilliant SYBR Green Kit (Stratagene, Amsterdam, Netherlands) on a Mx3000p cycler (Stratagene, Amsterdam, Netherlands). Cycling conditions were as followed: 10:00 min /95°C (activation), 00:30 sec/95°C (denaturation), 00:30 sec/60°C (annealing with fluorescence measurement), 00:30 sec/72°C (elongation).

***Quantitative determination of cyclic AMP (cAMP).*** For the determination of cAMP in cell supernatants we used the competitive cAMP ELISA parameter kit (R&D Systems, Minneapolis, MN) as described in the protocol by the manufacturer. Supernatants were isolated after one minute of incubation.

***RNA isolation and cDNA synthesis**.* 100,000 cells were seeded in six-well plates and grown until confluency. Total RNA was isolated using the Rneasy Midi Kit (Qiagen, Hilden, Germany). DNase I digest was performed with RNase free DNase Kit (Qiagen, Hilden, Germany) on-column. Prior to qPCR, total RNA contents were quantified photometrically at 260 nm. The cDNA was synthesized using the cDNA High Capacity Synthesis Kit (Applied Biosystems, Munich, Germany), as described in the manufactures manual.

***Secretory Activity**.* A volume of two mL cell suspension corresponding to 2,000,000 cells was used for each biological independent experiment and was incubated for 10 minutes with 2.5 mg coffee lyophililsate per mL PBS, histamine, single compounds or different combinations of all compounds at 37°C. The secretory activity was measured by the determination of the intracellular proton index (IPX). The fluorescence dye Carboxy-SNARF-AM (Invitrogen, Karlsruhe, Germany) was used for the measurement of the Intracellular pH. The method was validated. HGT-1 cells were loaded with 3 µM dye for 30 minutes on-ice. The intracellular pH was calculated referring to a calibration curve with 2 µM nigericin (PAA, Coelbe, Germany) treated HGT-1 cells in K+ clamp buffer consisting of 20 mM NaCl, 110 mM KCl, 1 mM CaCl2, 1 mM MgSO4, 18 mM D-Glucose and 20 mM HEPES that was set to different pH calibration points (6.8-8.2) by titration with NaOH. Then, the intracellular proton index (IPX) was calculated by log2 transformation of the intracellular proton concentration ratio between treated cells and control cells. The higher the IPX, the more protons remain in the cell, indicating a lower secretory activity. A more negative IPX stands for a higher secretory activity respectively.

***Ussing chambers experiments**.* To test how single substances impair the ion transport from the basolateral to the apical site of a HGT-1 monolayer we performed Ussing chamber experiments. Therefore, HGT-1 cells were cultivated on polyester membrane SNAP-well permeable supports with 0.4 µm pores (Sigma-Aldrich, Munich, Germany). The surface of the cultivated area was about 1.13 cm². The cells were grown until confluency confirmed by microscopic analysis and resistance measurement with an Endohm chamber (WPI, Berlin, Germany). The SNAP-well inserts were mounted into Ussing chambers (Easy Mount Chambers, Harvard Apparatus, March-Hugstetten, Germany) and bathed with Ringer's solution (Sigma-Aldrich, Munich, Germany) at 37°C and where gently bubbled with air under pH control. All tested substances were also resolved in Ringer's solution. The transepithelial potential difference was measured by a pair of Ag/AgCl electrodes, connected to a voltage clamp apparatus (KMSCl, Aachen, Germany). The current and voltage electrodes were placed to each site of the monolayer. The electrodes were inserted into an electrode tip and connected to the Ringer's solution by a 3M KCI-filled agar-bridge. Secretion was measured as current in the short-circuit modus and displayed as µA/cm2 (pulse duration, 0.2 s; pulse frequency, 1 Hz; pulse amplitude, 25 ms). Compounds were added to the apical compartment indicating the gastric lumen.

*Signal transduction assay.* The phosphorylation status of receptor tyrosine kinases and MAPK kinases were detected by enzyme linked immuno assays (ELISA). Total protein was isolated from HGT-1 cells after incubation with the tested substances for 10 minutes according to the manufacturer's protocol and lysis buffer provided. Total protein content was quantified photometrically at 595 nm using Bradford's reagent (Bio-Rad, Munich). The following ELISAs were used for determination of the phosphorylation status: EGFr-ELlSA Kit, ERK1/2 ELISA Kit (both Calbiochem/Merck, Nottingham, UK), Akt1 pathscan ELISA and ATF-2 pathscan ELISA (both Cell Signaling/New England Biolabs, Frankfurt a. M., Germany). Absorption was read out at 450 nm on a MRX platereader (Dynex, Berlin, Germany).

***Neural network data analysis**.* The neural networks for the three receptors HRH2, CHRM3 and SSTR2 were individually trained on a set of coffee recombinate data. The neural network software, NeuralWorks Predict (NeuralWare, Pittsburg, PA), was used with default settings with the exception of the use of cascaded variable selection and forced treatment of the input as continuously variable numerical data. The relevance of individual coffee components on gene expression in the neural networks is characterized by the frequency in which the particular component appears in the final population of the genetic algorithm that selects network variables. A maximum frequency of 1.00 for an input variable (coffee component) indicates that it is very important for the neural network and is always chosen.

***Statistical analysis**.* Statistical analysis was performed with Excel 2003 or SigmaStat (Systat Software GmbH, Erkrath, Germany). Data sets generated by qPCR and ELISA were transformed by logarithmic conversion to reach normality and are indexed as log2 ratio in the figures. Single comparisons between treated and control cells were done with the two-tailed Students t-test for equal variances unless otherwise stated. For time course analysis of gene expression we performed a one-way ANOVA with Holm-Sidak post-hoc analysis for parametric data sets and the Kruskal-Wallis test with Dunn's post-hoc analysis for non-parametric data sets. Numbers of replicates for each experiment are stated in the results part and figures. In each diagram the mean value of the independent biological experiment ± standard error (SE) is shown. Ussing chamber experiments were performed as single experiments and the originally recorded values are shown in the according diagrams.

***Regulation of gastric acid secretion related genes**.* The regulation of gastric acid secretion related genes is an important mean to determine the susceptibility of the parietal cells towards paracrine and endocrine hormone secretion. Furthermore, the induced synthesis of functional proteins may impair the secretory activity of the parietal cell. In this study, we investigated the time-dependent influence of six different compounds deriving from coffee beverages on the expression of the H+, K+-ATPase (*ATP4A),* the histamine receptor H2 (*HRH2*), the acetylcholine receptor M3 (*CHRM3)* and the somatostatin receptor 2 (*SSTR2*). The time-dependent expression was measured until the expression returned to control levels. One counter-regulation after prior up- or down-regulation was considered to be a physiological regulation.

***H+, K+-ATPase**.* The expression of the H+, K+-ATPase was mostly and significantly impaired by chlorogenic acid, caffeine, pyrogallol and N-methylpyridinium (one-way ANOVA, p≤0.01-0.001; n=5-6, Fig. 7 A) whereas catechol and C5HT did not significantly change the expression over the whole time-course (graphs not shown). Chlorgenic acid and caffeine increased the expression of the H+, K+-ATPase by almost two fold (CA: 0.91±0.17, CAFF: 0.94 ±0.24; post-hoc test, p≤0.05, Fig. 7 A) after 20 minutes. In the whole time-course of 30 minutes the expression change was still significant. In contrast, N-methylpyridinium and pyrogallol lead to a significant decrease of expression after 20 minutes (N-MP: -0.64 ±0.11, PYR: -0.46 ±0.07, post-hoc test, p≤0.05, Fig. 7 A). To elucidate synergistic or anti-synergistic effects of these compound at their peak expression maxima/minima, different combination thereof were examined for the impact on gene expression. Both pyrogallol and N-methylpyridinium were able to significantly compensate the up-regulating effect of chlorogenic acid and caffeine (two-tailed *t*-test, p≤0.01-0.001, n=4-6, Fig. 7 B) to N-MP/CA: -0.76 ±0.24, N-MP/CAFF: -0.46 ±0.05, N-MP/CA/CAFF: -0.21 ±0.22 and PYR/CA: -0.36 ±0.19, PYR/CAFF: -0.10 ±0.13, PYR/CA/CAFF: -0.27 ±0.15. Hereby, it was indicated that in N-methylpyridinium and pyrogallol dominate over the effects of chlorogenic acid and caffeine in more complex solution. Treatment of HGT-1 cells with physiological inducers of gastric acid secretion, such as histamine (0.74 ±0.16; post-hoc test, p≤0.05, graph not shown) and acetylcholine (0.62±0.19; post-hoc test, n=6, p≤0.05, graph not shown), resulted in a significant increase of expression after 20 and 15 minutes respectively. Interestingly, the treatment with somatostatin also significantly up-regulated the H+, K+-ATPase after 20 minutes 0.83 ±0.20 (post-hoc test, n=6, p≤0.05, graph not shown). Considering these regulation patterns due to physiologic compounds, it seems likely that up-regulation of the H+, K+-ATPase occurs as first response to treatment of HGT-1 cells. Thus, the subsequent counter-regulation as demonstrated for pyrogallol and N-methylpyridinium is likely more essential for regulation of gastric acid secretion.

*Histamine receptor H2.* Pro-secretory receptors are involved in the activation of parietal cells due to the binding of hormones and transmitters. The pro-secretory histamine receptor H2 and acetylcholine receptor M3 were examined for their time-dependent expression in HGT-1 cells. The H2 receptor was significantly regulated over the whole time-course by chlorogenic acid, catechol and C5HT (one-way ANOVA, p≤0.01-0.05, n=5-6, Fig. 7 C) whereas caffeine and N-methylpyridinium did not impair the expression of this receptor. Catechol (-0.54 ±0.23, post-hoc test; p≤0.05) and C5HT (-1.35 ±0.21; post-hoc test, p≤0.05) down-regulated the expression significantly. Chlorogenic acid significantly up-regulated H2 receptor expression (0.55 ±0.21, post-hoc test, p≤0.05) after 20 minutes. The combination C5HT with chlorogenic acid resulted in a significant up-regulation of the H2 receptor after five minutes (0.46 ±0.04, two-tailed *t*-test, p≤0.001, n=4-6, Fig. 7 D) compared to control cells and C5HT alone. Since down-regulation of the H2 receptor would not increase the susceptibility of parietals cells towards histamine, C5HT acid may support this effect in a complex solution. In combination with chlorogenic acid it would be abrogated, however. The combination of catechol and chlorogenic acid did not change the expression level significantly compared to catechol alone (Fig. 7 D). Histamine as physiological ligand did not regulate the H2 receptor significantly (result not shown).

***Acetylcholin receptor M3**.* The cholinergic M3 receptor was only shown to be significantly regulated by caffeine, pyrogallol and N-methylpyridinium (one-way ANOVA, p≤0.01-0.001, n=4-9, Fig. 8 A). Chlorogenic acid, C5HT and catechol did not influence the expression of the M3 receptor significantly (graphs not shown). Caffeine, pyrogallol and N-methylpyridinium compounds firstly lead to an up-regulation of the M3 receptor after five minutes, CAFF: 0.55 ±0.08, N-MP: 0.53 ±0.25, PYR: 1.14 ±0.14 (post-hoc test, p≤0.05, Fig. 8 A) whereas pyrogallol showed the most pronounced up-regulation of this receptor. However, following counter-regulation was significant for N-methylpyridinium (-0.76 ±0.26, post-hoc test, p≤0.05, Fig. 8 B) after 20 minutes and for pyrogallol (-0.70 ±0.21, post-hoc test, p.0.05, Fig. 8 B) after 15 minutes. Since we had not measured any adverse up- or down-regulation by certain compounds we did not further investigate the effect of combined compounds on this receptor. The expression of the M3 receptor was not regulated significantly by treatment with acetylcholine (result not shown).

***Somatostatin receptor 2**.* The anti-secretory somatostatin receptor 2 was significantly regulated by N-methylpyridinium, C5HT and catechol (one-way ANOVA, p≤0.01-0.001, n=4-9, Fig. 8 B). Catechol decreased the expression of the somatostatin receptor to -0.56 ±0.28 after 15 minutes (post-hoc test, p≤0.05, Fig. 8 B). Moreover, C5HT caused a tremendous decrease of receptor expression to -2,12 ±0.25 after five minutes (post-hoc test, p≤0.05, Fig. 8 B). In contrast, N-methylpyridinium enhanced the expression the anti-secretory somatostatin receptor by more than two-fold to 1.14 .0.15 after five minutes (post-hoc test, p≤0.05, Fig. 8 B). Treatment with somatostatin regulated this receptor to the same extent like N-methylpyridinium, but with a peak maximum after 20 minutes (1.14 ±0.31; post-hoc test, p≤0.05, graph not shown). Thus, we combined C5HT and N-methylpyridinium, which resulted in expression of the somatostatin receptor at control levels (1.14 ±0.31, Fig. 8 D). The expression level compared to treatment with N-methylpyridinium or C5HT alone was therefore significantly changed (two-tailed t-test, p≤0.05-0.001, n=4-6, Fig. 8 D).

***Full recombination of compounds**.* After we had characterized the effect of every single compounds and their combinations for the most pronounced time points, we tested the whole recombination of all of the six compounds together. Our findings for the H+, K+-ATPase were confirmed by the fact that the expression of the proton pump was neither increased nor decreased after 20 minutes. The whole time-course was still significant for regulation (one-way ANOVA, p≤0.05, n=5-6, Fig. 9 A), but the compensating effect of N-methylpyridinium and pyrogallol after 20 minutes was confirmed. The pro-secretory M3 receptor was shown to be down-regulated after 20 minutes by N-methylpyridinium. The recombination of all six compounds also significantly down-regulated the expression of this receptor after 20 minutes to -1.43 ±0.31 (one-way ANOVA, p≤0.001, n=6, Fig. 9 A; post-hoc test, p.0.05) which was much stronger than the effect of N-methylpyridinium alone. Therefore, a combined effect of N-methytpyridinium an pyrogallol is suggested. Furthermore, the treatment with the full recombination lacked the up-regulation of the M3 receptor after five minutes suggesting anti-synergistic effects of all compounds together. The anti-secretory somatostatin receptor was shown to be up-regulated by N-methylpyridinium and to be adversely regulated by C5HT. The full recombination confirmed our findings for C5HT/N-methylpyridinim counteracting to each other after five minutes. The expression level was slightly, but not significant above control levels. In contrast, the subsequent down-regulation of the somatostatin receptor took still place after 20 minutes as also shown for N-methylpyridinium alone (one-way ANOVA, p≤0.001, n=6, Fig. 9 A; post-hoc test, p≤0.05).

The histamine H2 receptor drew a completely different picture than shown for the single compounds. The strong down-regulating effect of C5HT was likely compensated due to chlorogenic acid as shown before (Fig. 7 C and D). However, after 10 minutes the expression of the H2 receptor was tremendously elevated to 1.36 ±0.37 (one-way ANOVA, p≤0.001, n=6, Fig. 9 A; post-hoc test, p≤0.05). Since our single compound experiments lack for an explanation for this effect, we set up an approach to find relationships between single compounds or combinations thereof and H2 receptor expression as well as signal transduction and functional aspects by a statistical modeling which is introduced in the next paragraph.

***Identification of combinative effects on receptor gene expression by computational analysis**.* Various combinations of the tested compounds were used for the evaluation of effects due to combination of single compounds by calculation of a neuronal network (Table 2). As most dominant compounds in a multi compound solution N-methylpyridinium, caffeine and chlorogneic acid were identified. N-methylpyridinium was considered to be an important factor in 94% of all calculated scenarios for the expression of the histamine H2 receptor. The highest relevance showed caffeine with 100% Pyrogallol and chlorogenic acid had a relevance of 71% and 79% whereas N-alkanoyl-5-hydroxytryptamides and catechol had a lower relevance of 50% and 29%. In total the calculated scenarios correlated with experimental data for the expression of the histamine H2 receptor with R=94%. The experimental results for the expression of the acetylcholine receptor M3 also had a correlation of 94% with the calculated model. Here, chlorogenic acid had the highest relevance with 100%. N-methylpyridnium was used in 91% of all calculated scenarios as an important factor in combination with other components. Caffeine had a relevance of 82%. Catechol (59%), N-alkanoyl-5-hydroxytryptamide (68%) and pyrogallol (23%) were of minor importance. The neuronal network that was calculated for the anti-secretory somatostatin receptor correlated still with an acceptable R-value of 80% with the experimental data. Apart from the N-alkanoyl-5-hydroxytryptamides (25%) all compounds showed a relevance above 83% (pyrogallol). The highest weight factor was calculated for chlorogenic acid and caffeine with 100%. N-methylpyridinium showed a relevance of 96% as same as catechol.

***Cyclic AMP concentration in cell lysates**.* Histamine H2 receptor signaling is strongly related to elevated cAMP levels due to activation of adenylatcyclase. Treatment with histamine significantly increased the cAMP concentration in HGT-1 cells to 71 ±17 fmol/mL (two-tailed *t*-test; p≤0.05; n=4; Fig. 10 A). Similar to histamine also caffeine (56 ±15 fmol/mL), pyrogallol (56 ±23 fmol/mL), catechol (74 ±27 fmol/mL), C5HT (40 ±1.0 fmol/mL) and N-methylpyridinium increased cyclic AMP levels in HGT-1 cells significantly (two-tailed t-test; p≤0.05-0.01; n=4; Fig. 10 A). In contrast, chlorogenic acid (12 ±1.9 fmol/mL) lead to significantly decrease cyclic AMP concentrations compared to control cells (two-tailed *t*-test; p≤0.05; n=4; Fig. 10 A). The full recombination of all single compounds also significantly increased the cyclic AMP concentrations to 46 ±7.1 fmol/mL compared to controls (23 ±3.4 fmol/mL) (two tailed *t*-test, p≤0.05; n=7; Fig. 9, C).

***Signal transduction**.* Induction of gastric acid secretion is multi-parametric. Changes at regulatory and functional level are mediated by intracellular signaling of kinases. Therefore, we examined whether three different kinases are activated by phosphorylation after treatment with roasted coffee constituents or histamine. Firstly, EGFr receptor tyrosine kinase phosphorylation after addition of histamine (1.32 +0.42), chlorogenic acid (2.22 ±0.30), caffeine (1.78 ±0.64) or C5HT (1.19 ±0.21) was significantly increased (p≤0.001-0.05; n=3-7; Fig. 10, B) whereas N-methylpyridinium did not change activation compared to control cells. Because EGFr and G-protein signaling subsequently can activate Akt1 kinase this pathway was also tested for its phosphorylation status. Surprisingly, Akt1 was only slightly activated by caffeine (0.48 ±0.30), pyrogallol (0.66 ±0.38), catechol (0.66 ±0.13) and C5HT (0.53 ±0.32) (two tailed t-test, p≤0.05; n=7; Fig. 10 B), but more significant by N-methylpyridinium (1.39 ±0.25) (two tailed *t*-test, p≤0.01; n=7; Fig. 10, B). Histamine induced gastric secretion is mainly progressed by cyclic AMP signaling. However, the chlorogenic acid did neither increase cyclic AMP levels nor activate Akt1 suggesting low secretory potential. At the same time activation of Akt1 by N-methylpyridinium has to be caused throughout induction of another receptor than EGFr. Further, we examined whether extracellular regulated kinases 1/2 (ERK1/2) show different phosphorylation levels after treatment with histamine or roasted coffee constituents. N-methylpyridinum (0.52 ±0.14) solely lead to significant activation of ERK1/2 (two tailed *t*-test, p≤0.01; n=3; Fig. 10, B). In contrast, treatment with histamine (-0.37 ±0.09), chlorogenic acid (-1.09 ±0.38), C5HT (-0.81 ±0.22) and caffeine (-0.30 ±0.13) even resulted in significantly decreased activation of ERK1/2 in comparison to control cells (two tailed *t*-test, p≤0.01-0.05; n=7; Fig. 10 B). The full recombination of all compounds, however, activated EGFr (2.19 ±0.59) and Akt1 (0.88 ±0.28), but not ERK1/2 (-0.33 ±0.14). This result indicates a predominant effect of other compounds that N-methylpyridinium in ERK1/2 signaling, which was previously found out to play an acute inhibitory role in gastric acid secretion.

***Transcription factor activation**.* Transcription factors have a crucial role in gene transcription. Since the genes for the H+, K+-ATPase alpha-subunit *(ATP4A)* and for the somatostatin receptor (*SSTR2*) exhibit cyclic cAMP responsive elements, we investigated the role of the transcription factor ATF-2 in stimulation of gastric acid secretion due to histamine, single compounds and a full recombination thereof. Only histamine (1.17 ±0.25), catechol (1.22 ±0.08) and N-methylpyridinium (0.84 ±0.23) significantly activated ATF-2. Solely caffeine caused a dephosphorylation that was significant below control cells (0.64 ±0.06) (two tailed *t*-test, p≤0.05-0.001; n=7; Fig. 10, C). The full recombination of all compounds lead to a significantly increased activation of ATF-2 compared to controls (two tailed *t*-test, p≤0.01; n=4; Fig. 9, B). Related to the measurements of cyclic AMP concentrations in HGT-1 cells, histaminic activation of parietal cells is indicated to be strongly linked to activation of ATF-2 as well as catechol and N-methylpyridinium. Moreover, ATF-2 dephosphorylation by chlorogenic acid is consistent with decreased cyclic AMP levels in HGT-1 cells. It is likely that the transcription of gastric acid secretion related genes is also activated du to other pathways and transcription factors than cyclic AMP signaling since caffeine and chlorogenic acid caused a strong increase in expression of the H+, K+-ATPase. However, N-methylpyridinium induced expression of the somatostatin receptor may be supported both by cyclic AMP and activation of ATF-2.

***Secretory potential**.* The intracellular proton index (IPX) was firstly measured to evaluate the stimulatory potential of the single compounds, the full recombination and of different recombinations that were lacking each one of the six compounds. The IPX provides information about the intracellular proton concentration compared to non-treated cells. A more negative IPX that control cells indicates a higher secretory activity assuming that the intracellular pH is temporarily increased by proton secretion. The extent of a pH change, however, is in the in the physiological range, but measurable. Histamine caused a strong secretory stimulation referred to an IPX of -1.15 ±0.04 (two tailed t-test, p≤0.01; n=4; Fig. 11 A). This finding was supported by Ussing chamber measurement where histamine enhanced secretion to 39 µA/cm² (Fig. 11 B) that was later inhibited by omeprazole, a specific inhibitor of the H+, K+-ATPase, and then returned to control levels (77 µA/cm², Fig. 11 B). The effect of chlorogenic acid was strongly anti-secretory. Both the measurement of a significantly positive IPX (0.17 ±0.02; two tailed *t*-test, p≤0.001; n=7; Fig. 11 A) and a decreased current in the Ussing chamber (-76 µA/cM2 , Fig. 11 C) suggest an inhibitory effect of chlorogenic acid at functional level *in vitro.* The recombination of all compounds lacking chlorogenic acid also indicated an anti-synergistic effect of chlorogenic acid in the full recombination since the IPX (0.02 ±0.01) was significantly decreased (two tailed *t*-test, p≤0.001; n=7; Fig. 11, A). In contrast, caffeine stimulated secretion by an IPX of -0.09 ±0.04 (two tailed *t*-test, p≤0.01; n=7; Fig. 11, A). The secretory activity was confirmed by an increase of the current to 16 µA/cm² (Fig. 11 C). N-methylpyridinium alone did not effect the IPX (Fig. 11 A) nor the current (graph not shown) at all. Therefore, if added to the basal compartment of the Ussing chamber in lead to an decrease of current from 16 µA/cm² to -5 µA/cm² (Fig. 11 C), if caffeine had been added to the apical compartment before. A combination of caffeine with N-methylpyridinium resulted in a decreased IPX of 0.03 ±0.06 which was not significant from the control level anymore, but neither from the effect of caffeine alone (result not shown). The strongest effect of recombination was found for catechol. The IPX of catechol alone indicated a stimulatory effect on HGT-1 cells (-0.04 ±0.02; two tailed *t*-test, p.0.05; n=7; Fig. 11, A). The recombination of all compounds lacking catechol turned out in a highly significant change to a positive IPX of 0.12 ±0.02 (two tailed *t*-test, p≤50.001; n=7; Fig. 11, A). As indicated with the IPX of catechol, which was still significant, but not as strong as histamine or caffeine, the current caused by apical treatment of HGT-1 cells with catechol was relatively smooth compared to stimulation with caffeine or histamine (Fig. 11 A, C, D). Thus, the effect of catechol is seemingly more synergistic than a single compound impact. C5HT was not tested to change the IPX significantly compared to control cells, however, C5HT alone showed a tendency to significance with a p-value of 0.07. This is supported by the measurement of current after apical C5HT treatment of HGT-1 cells, which resulted in a increased current of 70 µA/cm2 after addition (Fig. 11E). Finally, pyrogallol was tested to exhibit an anti-secretory ability with a significant positive IPX of 0.05 ±0.02 compared to control cells (two tailed *t*-fest, p≤0.05; n=7; Fig. 11, A). The measurement of current resulted in a decrease to -24 µA/cm2 after apical treatment of HGT-1 cells. The full recombination, however, did not show a significant change of IPX compared to control cells supporting the hypothesis of synergistic and anti-synergistic effects in multi-compound solutions.

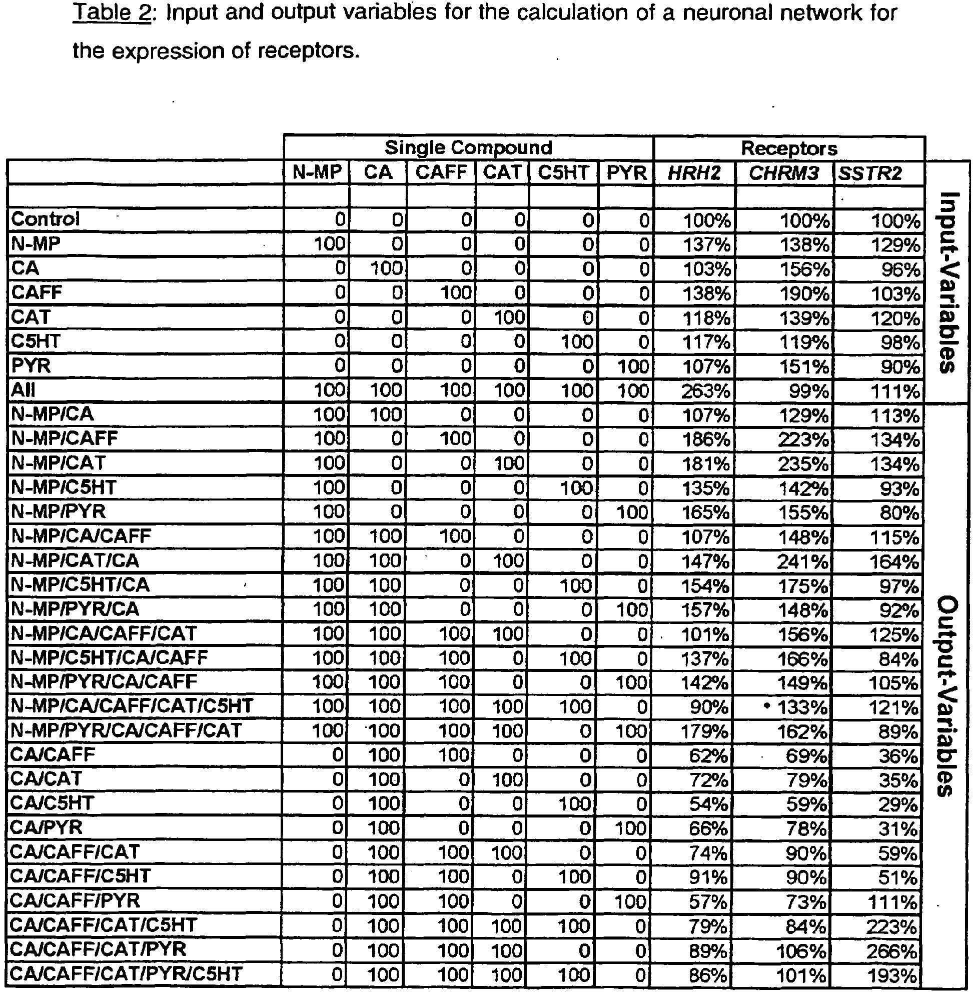

Neural network analysis of the gene expression of the HRH2, the CHRM3 and the SSTR2 receptors revealed that the receptor specific compound of least impact was catechol, pyrogallol and C5HT, respectively (Table 3). However, the data indicate a compound-specific regulation of the individual receptors since for the gene expression of HRH2 receptor, caffeine was most relevant, whereas the gene expression of the CHRM3 receptor was affected most by chlorogenic acid. For the SSTR2 receptor, chlorogenic acid and caffeine were equally important and reached also the highest frequency.

**Table 3: Relevance of individual coffee components in the neural networks for gene expression of the HRH2, CHRM3 and SST2 receptors. R is the linear correlation between the measured value and the neural network prediction.**

| | **HRH2** | **CHRM3** | **SSTR2** |
|---|---|---|---|
| NMP | 0.94 | 0.91 | 0.96 |
| CS | 0.79 | 1.00 | 1.00 |
| COFF | 1.00 | 0.82 | 1.00 |
| CAT | 0.29 | 0.59 | 0.96 |
| 5HT | 0.50 | 0.68 | 0.25 |
| PYR | 0.71 | 0.23 | 0.83 |
| | R = 0.94 | R = 0.94 | R = 0.80 |

All compounds were applied in their concentration like they occur in a regular coffee beverage. In our multi-parametric approach we examined the effect of these compounds on so far known genes, signals transducers and functional aspects of gastric acid secretion. In addition, we applied the neural network modeling in order to identify compounds of most importance for the gene expression of pro- and anti-secretory receptors. However, our findings for single compounds on the level of gene regulation were meeting, with one exception, the outcome of full recombination measurements. N-methylpyridinium and pyrogallol were shown, for the first time, to down-regulate the key player of gastric acid secretion, the H+, K+-ATPase, and to counteract towards caffeine and chlorogenic acid which up-regulated this gene by almost two-fold. In recombination experiments both N-methylpyridinium and pyrogallol compensated the up-regulating effect of caffeine and chlorogenic acid, so did also the full recombination of all compounds. The pro-secretory acetylcholine receptor M3 was up-regulated by pyrogallol, chlorogenic acid and N-methylpyridinium. N-methylpyridinium subsequently down-regulated this receptor after 20 minutes in the same way like the full recombination of all compounds effected the expression of this receptor. Additionally, pyrogallol was also down-regulating this receptor at 15 minutes after strong up-regulation at five minutes. A combined effect after 20 minutes is therefore likely. Additionally, the anti-secretory somatostatin receptor 2 was strongly down-regulated by C5HT which may result in reduced susceptibility of parietal cells to anti-secretory signaling *in vivo,* In contrast, N-methylpyridinium increased the expression of this receptor by more then two-fold. A combination of both compounds ended up in expression levels non-significant to controls. In the same way, the full recombination of all compounds regulated the anti-secretory somatostatin receptor.

Summarizing these results, N-methylpyridinium seemingly effected all important genes, except the histamine receptor, in an anti-secretory manner. Pyrogallol had equal effects on the H+, K+-ATPase and a co-effect with N-methylpyridinium on the acetylcholine receptor M3 is likely. Since C5HT strongly down-regulates the anti-secretory somatostatin receptor and compensates the up-regulating effect of N-methylpyridinium C5HT may support enhanced gastric acid secretion after coffee consumption *in vivo.* In contrast, C5HT down-regulated the expression of the histamine H2 receptor tremendously after five minutes whereas chlorogenic acid lead to an up-regulation after 15 minutes. The recombination of all compounds, however, showed a normal expression level after five minutes, but an increase of expression of more than 2-fold after 10 minutes. Here it remains unclear, why combined effects cause the up-regulated expression of the H2 receptor. The neuronal network predicted the highest relevance for the expression of the histamine H2 receptor for caffeine, chlorogenic acid and N-methylpyridinium. The various different combinations of single compounds revealed that an increasing complexity of solutions ended in different values of relevance than single compounds would indicate. Since the lowering effect of C5HT on the expression of the acetylcholine M3 receptor was even increased by co-incubation with chologenic acid. The single effect of chlorogenic acid was not as strong to indicate a dominant influence over C5HT. Nevertheless, the neuronal network identified chlorogenic acid with the highest relevance for the change in expression of the acetylcholine receptor M3 due to multi-compound solutions with 100%. As compound with the second highest relevance, N-methylpyridinium was identified with 91%. However, N-methylpyridinium showed a down-regulating effect on the expression of the acetylcholine receptor M3 as single compound. These findings are supported by high R-values for the correlation of the calculated scenarios and the experimental data. Because no single compound and neither the full recombination of all tested compounds showed a strong impact on the expression of the histamine H2 receptor or on the acetylcholine receptor M3 after 10 min, the neuronal network servers a powerful tool to identify synergistic and anti-synergistic effects.

In terms of signal transduction, the activation of EGFr, Akt1 and ERK1/2 showed a differentiated picture. EGFr is so far thought to enhance gastric acid secretion and may also be partially activated by histamine. Our findings supported this hypothesis. In the same way acted caffeine, chlorogenic acid and C5HT as well as the full recombination. Interestingly, in a previous study, a coffee beverage with high contents of chlorogenic acid and caffeine, but low N-methylpyridinium also activated EGFr, but a beverages with high N-methylpyridinium did not activate this receptor. Subsequent signaling of EGFr involves Akt1 and ERK1/2 signaling. Akt1 is supposed to increase the expression of the H+, K+-ATPase and to increase secretory activity. All compounds, except histamine and chlorogenic acid, activated Akt1 signaling. The same did the full recombination of all compounds. In opposite, ERK1/2 signaling, which is supposed to acutely inhibit gastric secretion, was solely activated by N-methylpyridinium. In addition, the full recombination reduced ERK1/2 signaling below control level. Cyclic AMP and a related transcription factor, ATF-2, were shown not to be specifically impaired by either one of the tested compound, although histamine increased cellular cyclic AMP concentrations and ATF-2 activation. N-methylpyridinium acted in the same way. Since the effect of N-methylpyridinium was rather anti-secretory, increased cAMP levels and activation of ATF-2 could be linked to increased expression of the anti-secretory somatostatin receptor. In addition, chlorogenic acid decreased cyclic AMP levels and did not activated ATF-2 and was furthermore associated with a decreased secretory activity of HGT-1 cells. The secretory activity was characterized by intracellular pH measurement and Ussing chamber experiments. Caffeine, catechol and C5HT turned out stimulate secretion whereas chlorogenic acid and pyrogallol lead to a decrease in secretion. N-methylpyridinium did not impair the intracellular pH or the secretion in Ussing chamber experiments at all. Therefore, it exhibited an counter-regulating effect on caffeine induced secretion.

To sum up our findings, the biological effect of a single compound can always be different than the effect of the same compound in complex solutions. Our findings support the hypothesis of synergistic and anti-synergistic interactions of different components.

### Example 3: Quantitative analysis of NMP and 5CHT in coffee samples

N-alkanoyl-5-hydroxytryptamids (C5HT) and N-methylpyridinium (NMP) were quantitatively analyzed in a series of coffees, having different roast degrees and different pre-treatments. It was noted that the concentration of NMP in the coffee drink increased with increasing roast degree. The concentration of C5HT in the coffee drink decreased with increasing roast degree. The decaffeinated and dewaxed coffees exhibited markedly lower C5HT-contents compared to the untreated ones.

***Quantitative analysis**.* N-alkanoyl-5-hydroxytryptamide (C5HT) and N-methylpyridinium (NMP) were quantified in the coffee samples. The sample codes were replaced by easily accessible abbreviations for improved allocation (Table 4).

**Table 4: Overview over the designation of the analyzed coffees**

| **Designation of raw coffee** | | **Roast degree** | **Abbreviation** |
|---|---|---|---|
| untreated | Columbia 50006 | 50, 80, 110 Skt | "untreated Col 5" |
| | Columbia 26902A | 50, 80, 110 Skt | "untreated Col 2" |
| decaffeinated | 26902 A+26831 A | 50, 80, 110 Skt | "decaffeinated 2+2" |
| dewaxed | Columbia 50006 | 50, 80, 110 Skt | "dewaxed Col 5" |
| | Columbia 26902 A | 50, 80 Skt | "dewaxed Col 2" |

For quantitative analysis of the N-alkanoyl-5-hydroxytryptamides (C5HT) an aliquot of the freshly prepared coffee drink was mixed with the deuterium-labeled internal standards and diluted with methanol after equilibration. Cooling and centrifugation resulted in a brown-colored precipitate and a yellowish supernatant which was injected into the HPLC-MS/MS system without further processing. Fig. 16 shows a representative analysis. The terms "Skt" and "scale parts" are used herein synonymously.

While the analyzed coffee drinks exhibited essentially the same tendencies concerning the concentration of NMP and C5HT in dependence on the roast degree (in scale parts), in the drink of the dewaxed Columbia 2 (labeled in Fig. 15) at a medium roast degree, a strongly increased (+60%) content of NMP was determined compared to the other samples. This coffee (dewaxed Col 2, 80 Skt) and its untreated counterpart were used for NMP-spiking-experiments.

From the quantitative data for each coffee sample a dimensionless factor was calculated, being the quotient of NMP-concentration (mg/l) and C5HT-concentration (µg/l) x 100. This proposed factor is intended to illustrate the relation of C5HT to NMP at a glance. Besides the present samples, the data for commercially available coffees which were generated in previous studies are shown in Fig. 16 for comparative reasons.

It can be seen on the basis of the proposed factor, that the dewaxed Columbia 2 that is roasted at 80 scale parts stands out from the comparative samples in an extreme manner. Here, an extraordinarily relation of the concentrations of NMP to C5HT has been reached, without having to put up with a very dark roast color.

***Doping of N-methylpyridinium to a coffee drink**.* Standardized coffee drinks were prepared from the samples "untreated Columbia 2, 80 Skt" and "dewaxed Columbia 2, 80 Skt". A N-methylpyridinium iodide standard solution was doped to the drink prepared from the dewaxed roast coffee, resulting in a NMP content of +100%, +200% and +300% relative to the intrinsic NMP content in the mixture. After lyophilisation, the samples were further processed

**Table 5: Overview over the NMP-concentrations of doped coffee drinks**

| **Sample** | **Addition NMP⁺ (mg/l)** | **final concentration NMP⁺ (mg/l)** |
|---|---|---|
| untreated Columbia 2 | - | 15.4 |
| dewaxed Columbia 2 | - | 27.5 |
| | 29.5 (+100%) | 57.0 |
| | 59.8 (+200%) | 87.3 |
| | 89.3 (+300%) | 116.8 |

### Example 4: Proton secretion in gastric cells in response to NMP and trigonelline

In the following different amounts of N-methylpyridinium were added to two selected coffees. Further, trigonelline as a precursor substance of N-methylpyridinium as a single substance was characterized with regard to its effect on proton secretion of human gastric cells *in vitro.*

***Cell Culture**.* The tumor cell line HGT-1 (human gastric tumor cell line) was used for *in vitro* experiments. The cells were isolated from a primary gastric tumor of a 60 years old man. The HGT-1 cell line (clone 6) was established by Dr. Laboisse (Laboratory of Pathological Anatomy, Nantes, France) in 1982. The cell line grows adherently and microscopically exhibits the rhombic morphology that is characteristic for epithelial cells.

The cell line is cultured in an incubator at 37°C and 5% CO₂. Glutamine-free "Dulbecco's modified Eagle Medium" is used as culture medium with addition of:
20% fetal bovine serum
2% penicillin/streptomycin
2% glutamine

To minimize the influence of physiologically active components of the fetal bovine serum, an equivalent medium is used for culturing 24 h prior to the conduction of an experiment, but without addition of the serum. Cultivation of the HGT-1 cells is conducted in cell culture flasks (75 cm²) or cell culture plates (6-well, 24-well, 6-SNAP-well). The number of cells used for the individual experiments, as well as further details concerning the conduction of the individual experiments are presented in the respective section in detail.

***Measuring secretion via intracellular pH.*** Measurement of intracellular pH is conducted by flow cytometry. The intracellular pH is subject to tight regulation between a pH of 6.8 and 7.4. Larger variations are compensated by the intracellular proteinate buffer. The intracellular pH can be used as an indicator of the transfer of H⁺ ions across the cell membrane. A net transport of H⁺ ions from intracellular to extracellular results in a short-term increase of the intracellular pH and decrease of the intracellular H⁺ ion concentration, respectively.

The experiments are conducted as follows. The HGT-1 cells are washed with pre-warmed PBS and subsequently scraped in 5 ml PBS and resuspended. The suspension is adjusted to 1x10⁶ vital cells per ml and 2 ml each of the cell suspension are put in FACS-tubes. Now the cells are centrifuged at 1000 rpm for 5 min, the supernatant discarded, the pellet resuspended in 2 ml each of the pre-warmed sample solution at 37°C and incubated at 37°C for 10 min. After a new centrifugation the sample solution is taken up, the cells resuspended in 2 ml of the previously prepared 3 µM SNARF-AM solution, and put on ice for 30 min. The dye-solution is removed by a new centrifugation and the cell pellet resuspended in 1 ml cold PBS. During FACS-measurement the samples are kept on ice. In order to establish a calibration curve, 500 µl each of the adjusted calibration buffer solution (20 mmol/L NaCl, 110 mmol/L KCl, 1 mmol/L CaCl₂, 1 mmol/L MgSO₄, 18 mmol/L D-glucose and 20 mmol/L HEPES) are placed in FACS-tubes together with 1 µl nigericin. For the calibration curve, three additional tubes are filled with the adjusted cell suspension and incubated in PBS. After the SNARF-AM staining the cells are resuspended in 20 µl cold PBS and 5 µl each thereof are put in the prepared calibration tubes. Measurement is conducted analogous to the samples.

The measurement results are put out as a ratiometric quotient of the median of the counts of the two emission maxima of 580 nm and 640 nm of the dye, and calculated to the intracellular pH using the calibration curve. For the display of the results, the intracellular proton index was developed. This is calculated by a log₂-transformation of the relative ratio of the intracellular H⁺-ion concentration of a treated sample to a non-treated control. The lower this index, the more protons were translocated.

***Measuring secretion via transepithelial current**.* For the measurement of ion currents over a monolayer of HGT-1 cells, a Ussing-chamber of the firm Harvard-Apparatus was used. For the recording of current and voltage, an interface of the firm KMSI was used. It is essentially composed of two compartments, the apical and the basal chamber. Between both chambers a tissue sample or cells that are cultivated on a special membrane can be clamped. During the experiments, the chamber can be tempered to 37°C by a thermostat. For the experimental assembly, HGT-1 cells are cultured on SNAP-well membrane inserts. 100 000 cells are plated per well for a experimental set-up. The integrity of the cell monolayer is ensured via the transepithelial resistance using a ENDOHM-chamber and microscopic control. The SNAP-well insert is integrated between both chambers via a special frame. During the measurement it is possible to apply certain currents or voltages via the interface via two voltage and two current electrodes each (AgCl-electrodes), which are placed on both sides of the SNAP-well insert. For the measurement of ion currents the short-circuit mode (I_{sc}) is selected. To this end, the voltage is clamped at 0 volt. Since an electric potential is built up over the cell monolayer by active and diffusion-dependent charge transfer, an exact amount of current is applied via the electrodes, to result in a potential of 0 mV. The frequency of applying current is chosen such that over a short time a build up of a potential can take place. Thus, the measured currents represent a measure for the transport events that are taking place. To determine the specificity of a transport event, certain inhibitors, for which the transporter or channel that is inhibited is known, are used. This way, the transporters or ion channels that are responsible for changes in the current curve after application of a substance of which the effect is to be determined, can be determined. For the experimental set up in the present study, the activity of the H⁺, K⁺-ATPase was of interest. Histamine is a physiological stimulus of this H⁺/K⁺-exchanger. A pharmacological inhibitor binding selectively to the functional subunit of this proton pump is omeprazol.

***Sample preparation**.* For the experimental set-up, Arabica Columbia 2, untreated, 80 scale parts (Col2) and Arabica Columbia 2**, dewaxed, 80 scale parts (Col2**) were selected. The coffees were brewed by standard methods. The Arabica Columbia** was subsequently enriched with 2.95 mg/100 ml, 5.8 ml/100ml or 8.93 mg/100ml N-methylpyridinium. The coffee drinks were lyophilised for the cell culture experiments. For the experiments, the coffees were used at a concentration of 2.5 mg/ml phosphate buffer.

For the characterization of trigonelline, the concentration was used in accordance with the "Lehrbuch der Lebensmittelchemie" (497 µM). Histamine was used in all experiments at a concentration of 1 mM.

All substances and coffee lyophylisates were prepared in phosphate buffer (PBS).

***Influence of enriched coffees on secretion**.* HGT-1 cells could be significantly stimulated by histamine compared to the control (-0.38±0.06; p≤0.001). The tested coffees exhibited entirely a weakened secretory potential compared to the histamine-stimulated cells. Col2 (-0.21±0.02) stimulated the secretory activity significantly stronger compared to Col2** (0.09±0.03) (p≤0.05). Col2** coffees, enriched with N-methylpyridinium, showed a weaker effect than Col2. 3-fold enrichment with N-methylpyridinium (+8.93 mg/100ml) of Col2** resulted to the significantly (p≤0.001) weakest secretory activity (0.19±0.03) compared to Col2**. However, enrichment of Col2** with 5.98/100ml N-methylpyridinium already resulted in a significantly weaker secretion (0.03±0.01) compared to Col** (p≤0.05) (Fig. 17).

The cell culture experiment with coffees that were enriched with N-methylpyridinium could confirm the inhibitory effect of N-methylpyridinium on the secretory potential of coffee drinks. Additionally, it could be shown that the treatment of Col2 resulted in a weaker secretory potential of the resulting product Col2** compared to the initial product.

***Influence of trigonelline on secretion**.* N-methylpyridinium is generated by the pyrolytic decarboxylation of trigonelline. Subsequently, the single substance effect of trigonelline on the secretory activity of HGT-1 cells is shown. Histamine stimulated the secretion significantly (-0.67±0.06; p≤0.001). Incubation with trigonelline resulted in a significantly reduced secretory activity compared to the control (0.46±0.04; p≤0.001) (cf. Fig. 18).

An experiment concerning the concentration-dependent effect of trigonelline showed a significant relation. A stepwise decrease of the inhibitory effect of trigonelline could be shown over a range of concentrations from 4970, 497, 49.7, 4.97 µM. The IPX was reduced from 0.7 after treatment of the cells with the highest concentration of 4870 µM to -0.4 after treatment with a concentration of 4.97 µM (Fig. 19).

The inhibiting effect of trigonelline on the secretion compared to the control could also be confirmed in a diffusion-chamber experiment. After application of 497 µM trigonelline solution, the short circuit current decreased from +2 µA/cm² to -25 µA/cm². In contrast, N-methylpyridinium as a single substance had no detectable effect on the short circuit current (Fig. 20).

## Claims

1. A method for producing a N-methylpyridinium (NMP)-enriched extract from trigonelline-containing organic material, the method comprising the steps of:
(a) dewaxing the trigonelline-containing organic material;
(b) roasting the trigonelline-containing organic material;
(c) treating the roasted trigonelline-containing organic material with hot water to obtain an aqueous extract; and
(d) adding NMP to the aqueous extract.

2. The method according to claim 1, wherein the roast degree of the trigonelline-containing organic material after the roasting in step (b) is at least 50 scale parts.

3. The method according to claim 1 or 2, wherein in step (d) at least 2.95 mg/100 ml NMP are added to the aqueous extract.

4. The method according to any one of claims 1 to 3, wherein the final NMP-enriched extract contains at least 23 mg/L NMP.

5. The method according to any one of claims 1 to 4, wherein the ratio NMP/N-alkanoyl-5-hydroxytryptamide (C5HT) x 100 in the final NMP-enriched extract is at least 100.

6. The method according to any one of claims 1 to 5, wherein the trigonelline-containing organic material is selected from the group consisting of coffee, in particular *Coffea arabica, Coffea canephora, Coffea spp.,* and *Psilanthus spp.,* members of the *Fabaceae,* in particular *Pisum sativum, Glycine max, Phaseolus vulgaris, Lens culinaris, Cicer arietinum,* and *Trigonella foenum-graecum,* members of the *Chenopodiaceae,* in particular *Chenopodium quinoa,* and members of the *Poaceae,* in particular *Avena sativa.*

7. The method according to any one of claims 1 to 6, wherein the trigonelline-containing organic material is *Coffea arabica, provenience Columbia* or *Coffea arabica, provenience Brazil.*

## Patentansprüche

1. Verfahren zur Herstellung eines mit N-Methylpyridinium (NMP) angereicherten Extrakts aus Trigonellin enthaltendem organischen Material, wobei das Verfahren die Schritte umfaßt:
(a) Entwachsen des Trigonellin enthaltenden organischen Materials,
(b) Rösten des Trigonellin enthaltenden organischen Materials,
(c) Behandeln des gerösteten Trigonellin enthaltenden organischen Materials mit heißem Wasser, um einen wässrigen Extrakt zu erhalten, und
(d) Zugeben von NMP zu dem wässrigen Extrakt.

2. Verfahren nach Anspruch 1, wobei der Röstgrad des Trigonellin enthaltenden organischen Materials nach dem Rösten in Schritt (b) mindestens 50 Skalenanteile beträgt.

3. Verfahren nach Anspruch 1 oder 2, wobei in Schritt (d) mindestens 2,95 mg/100 ml NMP zu dem wässrigen Extrakt zugegeben werden.

4. Verfahren nach einem der Ansprüche 1 bis 3, wobei der fertige mit NMP angereicherte Extrakt mindestens 23 mg/L NMP enthält.

5. Verfahren nach einem der Ansprüche 1 bis 4, wobei das Verhältnis NMP/N-Alkanoyl-5-hydroxytryptamid (C5HT) x 100 in dem fertigen mit NMP angereicherten Extrakt mindestens 100 beträgt.

6. Verfahren nach einem der Ansprüche 1 bis 5, wobei das Trigonellin enthaltende organische Material aus der Gruppe, bestehend aus Kaffee, insbesondere *Coffea arabica, Coffea canephora, Coffea spp.* und *Psilanthus spp.;* Mitgliedern der *Fabaceae,* insbesondere *Pisum sativum, Glycine max, Phaseolus vulgaris, Lens culinaris, Cicer arietinum* und *Trigonella foenum-graecum;* Mitgliedern der *Chenopodiaceae,* insbesondere *Chenopodium quinoa;* und Mitgliedern der *Poaceae,* insbesondere *Avena sativa,* ausgewählt ist.

7. Verfahren nach einem der Ansprüche 1 bis 6, wobei das Trigonellin enthaltende organische Material *Coffea arabica, Provenienz Kolumbien* oder *Coffea arabica, Provenienz Brasilien* ist.

## Revendications

1. Procédé de production d'un extrait enrichi en N-méthylpyridinium (NMP) provenant d'un matériau organique contenant de la trigonelline, le procédé comprenant les étapes consistant à :
(a) éliminer les cires du matériau organique contenant de la trigonelline ;
(b) torréfier le matériau organique contenant de la trigonelline ;
(c) traiter le matériau organique contenant de la trigonelline torréfié avec de l'eau chaude pour obtenir un extrait aqueux ; et
(d) ajouter du NMP à l'extrait aqueux.

2. Procédé selon la revendication 1, dans lequel le degré de torréfaction du matériau organique contenant de la trigonelline après la torréfaction dans l'étape (b) est d'au moins 50 parties d'échelle.

3. Procédé selon la revendication 1 ou 2, dans lequel, dans l'étape (d), au moins 2,95 mg/100 ml de NMP sont ajoutés à l'extrait aqueux.

4. Procédé selon l'une quelconque des revendications 1 à 3, dans lequel l'extrait enrichi en NMP final contient au moins 23 mg/l de NMP.

5. Procédé selon l'une quelconque des revendications 1 à 4, dans lequel le rapport NMP/N-alcanoyl-5-hydroxytryptamide (C5HT) x 100 dans l'extrait enrichi en NMP final est d'au moins 100.

6. Procédé selon l'une quelconque des revendications 1 à 5, dans lequel le matériau organique contenant de la trigonelline est choisi dans le groupe constitué de cafés, en particulier *Coffea arabica, Coffea canephora, Coffea spp.* et *Psilanthus spp.,* membres de la famille des *Fabaceae,* en particulier *Pisum sativum, Glycine max, Phaseolus vulgaris, Lens culinaris, Cicer arietinum et Trigonella foenum-graecum,* membres de la famille des *Chenopodiaceae,* en particulier *Chenopodium quinoa,* et des membres de la famille des *Poaceae,* en particulier *l'Avena sativa.*

7. Procédé selon l'une quelconque des revendications 1 à 6, dans lequel le matériau organique contenant de la trigonelline est le *Coffea arabica,* en provenance de *Colombie* ou le *Coffea arabica,* en provenance du *Brésil.*
